# EUROPEAN PATENT SPECIFICATION

(11) **EP 3 193 920 B1**
(45) Date of publication and mention of the grant of the patent: **19.02.2020**
(21) Application number: 15763592.1
(22) Date of filing: 16.09.2015
(51) Int. Cl.: A61K 39/02, A61K 35/68, C12N 9/12, A61K 39/012, A61K 39/00, C12N 1/10, C12N 1/36, C07F 9/10

(54) **GENETICALLY MODIFIED COCCIDIAN PARASITES USEFUL AS VACCINES**
GENETISCH MODIFIZIERTE KOKZIDIENPARASITEN ALS IMPFSTOFFE
PARASITES DE COCCIDIES GÉNÉTIQUEMENT MODIFIÉS UTILES COMME VACCINS

(30) Priority: 16.09.2014 EP 14185015
(43) Date of publication of application: 26.07.2017
(73) Proprietor: Humboldt-Universität zu Berlin, 10099 Berlin (DE); Universiteit Utrecht Holding B.V., 3584 CM Utrecht (NL)
(72) Inventor: GUPTA, Nishith, 10115 Berlin (DE); ARROYO-OLARTE, Ruben, 10115 Berlin (DE); LUCIUS, Richard, 10115 Berlin (DE); BROUWERS, Jos, 3584 CM Utrecht (NL); HELMS, J. Bernd, 3584 CM Utrecht (NL); DUNAY, Ildiko, 10965 Berlin (DE)
(74) Representative: De Vries & Metman
(86) International application number: PCT/EP2015/071168
(87) International publication number: WO 2016/042008

(56) References cited:
- US-A1- 2011 250 265
- RAMESH VERMA ET AL: "Development of Toxoplasma gondii vaccine", HUMAN VACCINES & IMMUNOTHERAPEUTICS, vol. 9, no. 2, 1 February 2013 (2013-02-01), pages 291-293, XP055170485, ISSN: 2164-5515, DOI: 10.4161/hv.22474
- Erich Baer ET AL: "Phosphatidylthreonines I. SYNTHESIS OF DISTEAROYL-L-~~-GLYCERYLPHOSPHOSPHORYL-L-T HREONINE*", , 1 May 1962 (1962-05-01), XP055169972, Retrieved from the Internet: URL:http://www.jbc.org/content/237/5/1449. full.pdf#page=1&view=FitH [retrieved on 2015-02-16]
- P. T. IVANOVA ET AL: "Identification of atypical ether-linked glycerophospholipid species in macrophages by mass spectrometry", THE JOURNAL OF LIPID RESEARCH, vol. 51, no. 6, 30 November 2009 (2009-11-30), pages 1581-1590, XP055169910, ISSN: 0022-2275, DOI: 10.1194/jlr.D003715
- J. MITOMA ET AL: "Occurrence of an Unusual Phospholipid, Phosphatidyl-L-threonine, in Cultured Hippocampal Neurons: EXOGENOUS L-SERINE IS REQUIRED FOR THE SYNTHESIS OF NEURONAL PHOSPHATIDYL-L-SERINE AND SPHINGOLIPIDS", JOURNAL OF BIOLOGICAL CHEMISTRY, vol. 273, no. 31, 31 July 1998 (1998-07-31), pages 19363-19366, XP055169977, ISSN: 0021-9258, DOI: 10.1074/jbc.273.31.19363
- ZIQIANG GUAN ET AL: "Structural characterization of the polar lipids ofNT. Further evidence for a novel anaerobic biosynthetic pathway to plasmalogens", BIOCHIMICA AND BIOPHYSICA ACTA. MOLECULAR AND CELL BIOLOGY OF LIPIDS, ELSEVIER, AMSTERDAM, NL, vol. 1811, no. 3, 21 December 2010 (2010-12-21), pages 186-193, XP028135649, ISSN: 1388-1981, DOI: 10.1016/J.BBALIP.2010.12.010 [retrieved on 2010-12-30]
- DATABASE UniProt [Online] 16 October 2013 (2013-10-16), "SubName: Full=Putative phosphatidylserine synthase {ECO:0000313|EMBL:EPR64538.1};", XP002736170, retrieved from EBI accession no. UNIPROT:S7WJT7 Database accession no. S7WJT7
- DATABASE UniProt [Online] 16 October 2013 (2013-10-16), "SubName: Full=Phosphatidylserine synthase, putative {ECO:0000313|EMBL:EPT28665.1};", XP002736171, retrieved from EBI accession no. UNIPROT:S8GA55 Database accession no. S8GA55
- GUPTA N ET AL: "Selective disruption of phosphatidylcholine synthesis of Toxoplasma gondii markedly arrests parasite growth.", ABSTRACTS OF THE INTERSCIENCE CONFERENCE ON ANTIMICROBIAL AGENTS AND CHEMOTHERAPY, vol. 44, October 2004 (2004-10), page 450, XP009182669, & 44TH INTERSCIENCE CONFERENCE ON ANTIMICROBIAL AGENTS AND CHEMOTHERAPY; WASHINGTON, DC, USA; OCTOBER 30 -NOVEMBER 02, 2004 ISSN: 0733-6373
- V. SAMPELS ET AL: "Conditional Mutagenesis of a Novel Choline Kinase Demonstrates Plasticity of Phosphatidylcholine Biogenesis and Gene Expression in Toxoplasma gondii", JOURNAL OF BIOLOGICAL CHEMISTRY, vol. 287, no. 20, 11 May 2012 (2012-05-11) , pages 16289-16299, XP055170471, ISSN: 0021-9258, DOI: 10.1074/jbc.M112.347138

## Description

The invention is set out in the appended set of claims. The embodiments and/or examples of the following description which are not covered by the appended claims are not considered to be part of the present invention. The present invention relates to coccidian parasites wherein expression of phosphatidylthreonine synthase (PTS) is disrupted. Also disclosed are a polynucleotide comprising a nucleotide sequence encoding a phosphatidylthreonine synthase (PTS), a phosphatidylthreonine synthase (PTS) enzyme, and a phosphatidylthreonine (PtdThr) lipid.

Coccidian parasites, having the expression of PTS disrupted as described herein, are useful as vaccines. The phosphatidylthreonine synthase enzyme and the nucleotide encoding sequences thereof as well as the phosphatidylthreonine phospholipid can find use in diagnostic methods and diagnostic kits or in vaccine and drug development applications.

Coccidian parasites, such as *Toxoplasma, Eimeria,* and *Neospora* species, are unicellular pathogens, which pose a major socioeconomic burden worldwide. *Toxoplasma* (e.g. *Toxoplasma gondii*) is a widespread parasite infecting humans as well as nearly all other warm-blooded organisms. The parasite is a substantial threat to domestic animals of food and economic value (e.g. sheep, and pig) as well as to human health. The parasite causes cerebral and ocular toxoplasmosis in immunosuppressed individuals and in neonates. It can also inflict congenital disease in humans and animals leading to spontaneous abortions and fetal abnormalities. Similarly, *Eimeria* (e.g. *Eimeria tenella*) and *Neospora* (e.g. *Neospora caninum*) are major concerns for the poultry industry (causing e.g., diarrhea in chickens) and for the cattle industry (causing e.g., abortions in cows), respectively. Similar to *Toxoplasma, Neospora* parasites also form tissue cysts in the nervous system, which cannot be cured by drugs available, and thus persist for the entire life of the infected host. These cysts can reactivate to cause potentially fatal acute infection upon decline in immune response such as by HIV-AIDS infection or during organ transplantations or just by aging in elderly population.

Thus, there is a clear need for preventive methods to tackle coccidian parasite infections, in particular the yet-incurable cyst (chronic) infection.

A commercial live vaccine against Toxoplasma is available (Toxovax), which comprises a live-attenuated S48 strain of *Toxoplasma gondii* (Buxton, D. (1993), Toxoplasmosis: the first commercial vaccine, Parasitology today (Personal ed.), 9(9), 335-7 (review); Verma R. and Khanna P. (2013) Development of Toxoplasma gondii vaccine: A global challenge, Hum Vaccin Immunother., 2013 Feb;9(2):291-3 (review)). This vaccine is used in veterinary applications. However, it has several disadvantages: it is expensive, causes side effects, has a short shelf-life, and is genetically uncharacterized. Also, it is not suitable for human use because it is susceptible to reverting to its pathogenic virulence.

The inventors have identified a novel enzyme (phosphatidylthreonine synthase, PTS) expressed in coccidian parasites which is absent in all mammalian hosts, and which produces an exclusive membrane lipid known as phosphatidylthreonine (PtdThr). It is noteworthy that PtdThr has been previously reported as a rare and notably minor PtdSer analog in certain mammalian cells and in selected prokaryotes (J. Mitoma, T. Kasama, S. Furuya, Y. Hirabayashi, Occurrence of an unusual phospholipid, phosphatidyl-L-threonine, in cultured hippocampal neurons. Exogenous L-serine is required for the synthesis of neuronal phosphatidyl-L-serine and sphingolipids., The Journal of Biological Chemistry 273, 19363-19366 (1998); P. T. Ivanova, S. B. Milne, H. A. Brown, Identification of atypical ether-linked glycerophospholipid species in macrophages by mass spectrometry., Journal Of Lipid Research 51, 1581-1590 (2010); F. D. Muller, S. Beck, E. Strauch, M. W. Linscheid, Bacterial predators possess unique membrane lipid structures., Lipids 46, 1129-40 (2011); L. Heikinheimo, P. Somerharju, Translocation of phosphatidylthreonine and -serine to mitochondria diminishes exponentially with increasing molecular hydrophobicity., Traffic 3, 367-77 (2002)), occasionally with rather detrimental effect on cell physiology (J. Mitoma, T. Kasama, S. Furuya, Y. Hirabayashi, Occurrence of an unusual phospholipid, phosphatidyl-L-threonine, in cultured hippocampal neurons. Exogenous L-serine is required for the synthesis of neuronal phosphatidyl-L-serine and sphingolipids., The Journal of Biological Chemistry 273, 19363-19366 (1998)).

It was shown that the base-exchange type phosphatidylserine synthase (PSS) in mammalian cells, normally using serine as its primary substrate, could also produce PtdThr as a byproduct under serine-deprived conditions. Under normal conditions, eukaryotic cells do not produce PtdThr in any detectable amounts. In contrast, the inventors reveal a surprisingly abundant and natural occurrence of PtdThr in a widespread eukaryotic pathogen, such as *T. gondii* and *E. tenella.*

The inventors have also found that the disruption of PTS abrogates the de novo synthesis of PtdThr, and impairs the asexual life cycle of coccidian parasites (e.g. T. *gondii*). In particular, the inventors have found that so modified parasites are avirulent and confer protection against parasite-inflicted diseases, such as acute and chronic toxoplasmosis in mice. The absence of PTS and of PtdThr being produced in mammalian cells means that the PTS-disrupted parasites cannot obtain sufficient PtdThr from the host cells to compensate for their PtdThr deficiency. This makes coccidian parasites with disrupted PTS expression rather avirulent and useful as vaccines, particularly for the farm and food animals, often infected by *T. gondii.*

Accordingly, the present invention relates to a coccidian parasite selected from *Toxoplasma, Neospora* and *Eimeria* species wherein the expression of phosphatidylthreonine synthase (PTS) enzyme is disrupted thereby eliminating the synthesis of phosphatidylthreonine (PtdThr). Suitable coccidian parasites include *Toxoplasma, Neospora* and *Eimeria* species. *Toxoplasma gondii* (also referred to as *T. gondii* or *Tg*), *Neospora canium* (also referred to as *N. canium* or Nc) and *Eimeria tenella* (also referred to as *E. tenella* or *Et*) may be particularly mentioned. *Toxoplasma gondii may* be of particular interest.

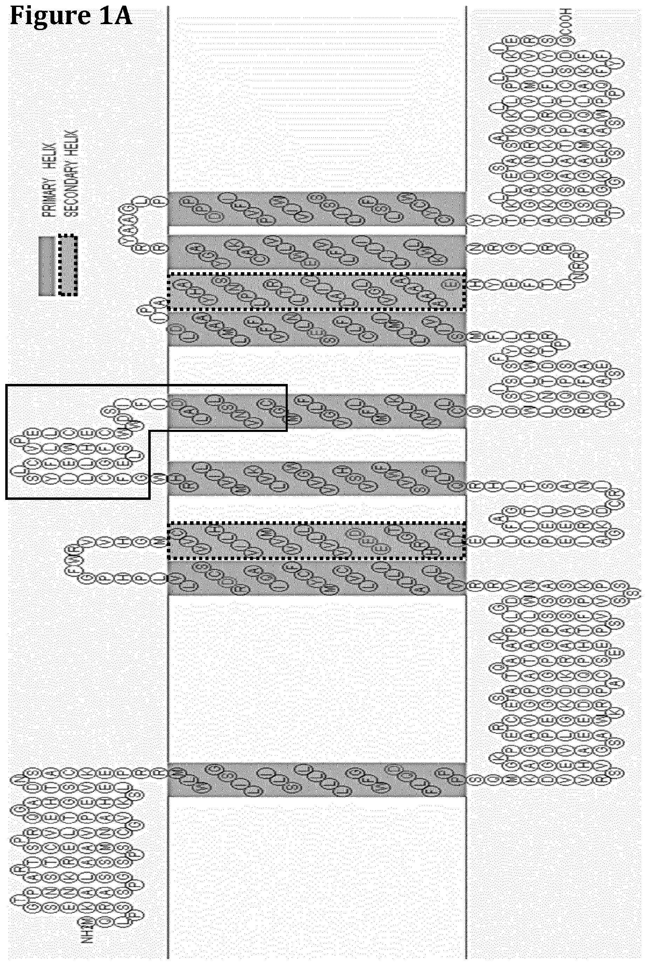

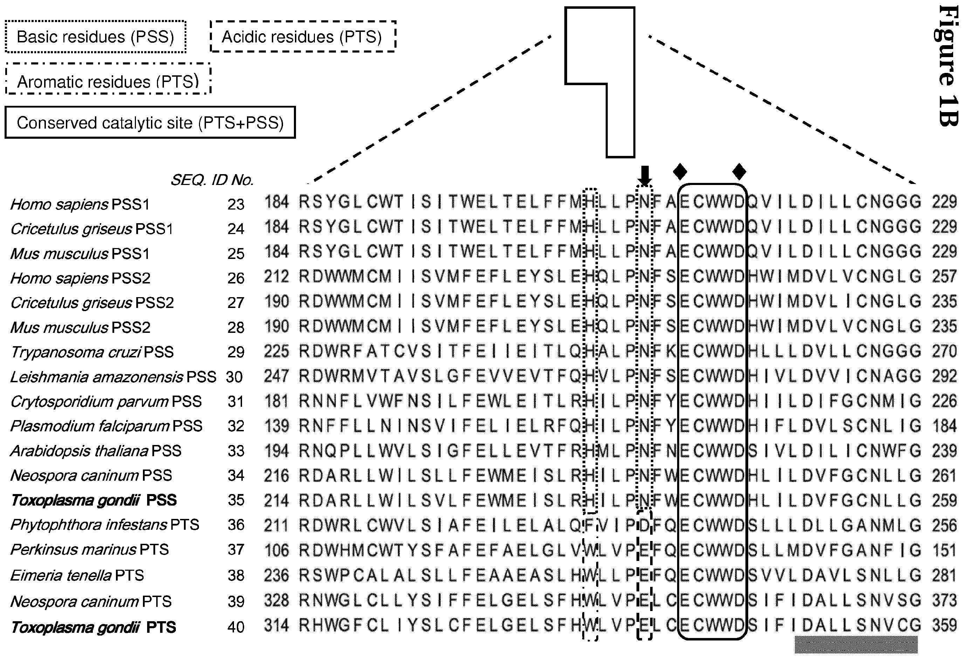

PTS is an enzyme expressed in coccidian parasites, which produces phosphatidylthreonine (PtdThr). This enzyme is distinct from the closely related and more ubiquitous phosphatidylserine synthase (PSS), which is also present in coccidian parasites. PSS produces phosphatidylserine (PtdSer) instead of phosphatidylthreonine (PdtThr). PTS and PSS share the same catalytic site, of sequence ECWWD, but differ in many other residues in the catalytic domain (Figure 1). Of note is the fact that distinct asparagine, histidine and cysteine residues are strictly conserved in all PSS orthologs including in *Tg*PSS, however not in *Tg*PTS, which contains substitutions to glutamate, tryptophan and serine at the equivalent positions (Figure 1).

The gene sequence encoding PTS, also referred to as PTS gene, is provided in SEQ ID No. 1 for *Toxoplasma gondii,* in SEQ ID No. 3 for *Neospora caninum* and in SEQ ID No. 5 for *Eimeria tenella.* The corresponding encoded amino acid sequence is provided in SEQ ID No. 2 for *Toxoplasma gondii,* in SEQ ID No. 4 for *Neospora caninum* and in SEQ ID No. 6 for *Eimeria tenella.*

PtdThr is a glycerophospholipid characterized by having a threonine bound to the phosphatidyl group via the OH group of threonine. Generally, PTS may produce a mixture of phosphatidylthreonine lipid species, whose structures may vary in the nature of the acyl groups attached to glycerol.

The structure of a phosphatidylthreonine (PtdThr) as described herein may be depicted by formula (I) 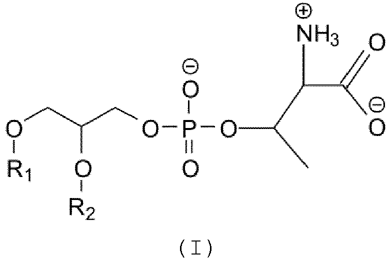 wherein R₁ and R₂ are independently selected from saturated and/or unsaturated acyl groups having from 8 to 46 carbon atoms, in particular from 10 to 36, more in particular from 14 to 26, and yet more in particular from 16 to 24 carbon atoms. Unsaturated acyl groups may have at least one carbon-to-carbon double bond, particularly 1 to 8, more in particular 1 to 6, and even more in particular 1 to 4 carbon-to-carbon double bonds. In several embodiments, R₁ and R₂ may be independently selected from unsaturated acyl groups having from 16 to 24 carbon atoms having 1 to 4 carbon-to-carbon double bonds. In particular embodiments, R₁ and R₂ may be independently selected from C20:1 and C20:4 acyl groups, more in particular, R₁ and may be an acyl group of formula CH₃-(CH₂)₇-(CH=CH)-(CH₂)₉-CO- and R₂ may be an acyl group of formula CH₃-(CH₂)₄-(CH=CH-CH₂)₄-(CH₂)₂-CO-.

In coccidian parasites as described herein, the expression of phosphatidylthreonine synthase (PTS) is disrupted. As indicated above, the disruption of PTS expression has been found to reduce or eliminate the synthesis of phosphatidylthreonine (PtdThr). The expression of PTS may be disrupted, e.g. by inactivating or deleting the gene encoding for the PTS enzyme. For instance, a coccidian parasite as described herein may be genetically modified.

Accordingly, in several aspects, the instant invention is directed to a method for preparing a genetically modified coccidian parasite, which comprises disrupting the expression of endogenous phosphatidylthreonine synthase (PTS) enzyme in a coccidian parasite selected from *Toxoplasma, Neospora* and *Eimeria* species by inactivating or deleting the corresponding PTS-encoding gene.

In the context of the invention, inactivation or deletion of a gene may be modification of a gene encoding a desired polypeptide (i.e. PTS) and/or a gene encoding a polypeptide involved in production of a primary or secondary metabolite by the parasite. In principle, eliminating the activity of the encoded protein can be done by targeted inactivation of the gene encoding PTS. The gene can be removed in its entirety. However, as an alternative also the deletion of part of the gene might result in elimination of the activity of the encoded protein. For instance, the nucleotides encoding for the catalytic site of PTS may be removed or replaced, e.g. by a distinct nucleotide sequence, which lacks the nucleotides encoding for the catalytic site of PTS. The resulting genetically modified parasite will have the expression of PTS ablated. Consequently, the production of PtdThr can be eliminated. The catalytic site of PTS is highly conserved and comprises the amino acid sequence ECWWD. In particular, this catalytic sequence is found on positions 342-346 of the amino acid sequence of PTS for *T. gondii* and on positions 264-268 for *E. tenella* and 356-360 for *N. caninum.* The regions flanking the nucleotide sequence encoding for the catalytic site of PTS can be identified by standard DNA sequencing.

Alternatively, or additionally, nucleotide sequences responsible for the regulation or expression of the PTS-expressing gene, such as promoters enhancers and translational initiator sites can be modified or removed. Another way to influence the activity of PTS might be the modification of transport signals, if needed, or the introduction of anti-sense RNA.

Chromosomal modification is preferred since chromosomal modification will ensure a stable distribution of the functionality of the gene over the progeny cells. Deletion or disruption of a desired functionality in the chromosome (e.g. deletion or disruption of the sequence encoding PTS or encoding the catalytic site of PTS) can be done with non-homologous as well as with homologous recombination. Homologous recombination is preferred, as it opens the opportunity to introduce, to remove, or to simultaneously introduce and remove a functionality of choice. In particular, the gene expressing PTS may be disrupted by double homologous recombination.

When homologous recombination is intended, the transfected DNA constructs contain a DNA sequence that is homologous to a genomic target sequence of the specific parasite to be engineered. The skilled person will understand that 100% identity is not required to obtain homologous recombination. A percentage identity of 80%, preferably 90%, more preferably 95%, 98% or 99% will also suffice. Generally, the DNA sequence of interest to be inserted in the chromosome by homologous recombination is flanked by homologous sequences of a sufficient length to enable homologous recombination. Such a length may be at least about 300 bp in *T. gondii,* for instance between 300 and 3000 bp, depending on the parasite strains in use.

For the purpose of the present invention, the degree of identity between two amino acid sequences refers to the percentage of amino acids that are identical between the two sequences. The degree of identity is determined using the BLAST algorithm, which is described in Altschul, et al., (Journal of Molecular Biology, vol. 215, pp. 403-410 1990). Software for performing BLAST analyses is publicly available through the National Center for Biotechnology Information (http://www.ncbi.nlm.nih.gov/). The default settings for Blastp algorithm parameters are Expect threshold of 10, Word size of 3, Max matches in a query range of 0, Matrix is BLOSUM62, Gap Costs Existence of 11 and Extension of 1, Compositional adjustments at Conditional compositional score matrix adjustment.

For the purpose of the present invention, the degree of identity between two nucleotide sequences refers to the percentage of nucleotides that are identical between the two sequences. The degree of identity is determined using the BLAST algorithm, which is described in Altschul, et al., (Journal of Molecular Biology, vol. 215, pp. 403-410 1990). Software for performing BLAST analyses is publicly available through the National Center for Biotechnology Information (http://www.ncbi.nlm.nih.gov/). The default settings for Blastn algorithm parameters are Expect threshold of 10, Word size of 28, Max matches in a query range of 0, Match/Mismatch Scores of 1, -2, Gap Costs at Linear.

The sequences identifying the PTS genes in *T*. *gondii, N. caninum* and *E. tenella,* do not need to be 100% identical in order to modify the gene of interest by genetic engineering. In particular, the expression of PTS may be disrupted by inactivating or deleting a nucleotide sequence encoding an amino acid sequence of SEQ ID No. 2 (*Tg*PTS), SEQ ID No. 4 (*Nc*PTS) or SEQ ID No. 6 (*Et*PTS), or an homologous amino acid sequence (e.g. from other Eimeria or Neospora species) that displays a degree of identity of at least 30%, in particular at least 50%, more in particular at least 75%, even more in particular at least 90 %, yet more in particular at least 95 % and more in particular at least 99%.

Furthermore, the sequences in related parasites from other species or in parasites of particular strains might deviate from these sequences. However, making use of the PTS-expressing genes, sequences homologous to these genes, which have the same functionality, can be identified by those skilled in the art. Corresponding plasmid constructs can be prepared for performing homologous recombination in these strains. Thus, even if deviations from the sequences of the above-identified genes exist in a certain strain, homologous proteins can be identified using standard computational and wet-lab methods. For instance, the PTS activity of a given protein can be determined as explained below.

The parasites according to the present invention can be prepared using technologies known in the art. In particular, introducing DNA into parasites by electroporation (e.g. using a transfection instrument) may achieve transformation of coccidian parasites. Electroporation involves a high-voltage discharge passed through, e.g. a suspension containing coccidian parasites (for example *Toxoplasma, Neospora* or *Eimeria* species) and a suitable plasmid DNA comprising the desired functionality and/or homologous DNA sequences specific to the parasite in consideration.

The disruption of the PTS activity in a coccidian parasite as described herein may be tested by identifying the reduction or elimination of PtdThr production in the disrupted parasite when compared to the non-disrupted parasite (also referred to as wild-type or parental strain). In particular, lipid analyses may be used. For instance, the disrupted and the wild-type parasite strains may be grown in a suitable host (e.g. in human foreskin fibroblast (HFF) cells). The parasites can then be released from the host (e.g. using a syringe or by natural host-cell lysis by the parasite) and the lipids may be extracted from the parasites according to Bligh-Dyer (Bligh, E. G. & Dyer, W. J. "A rapid method of total lipid extraction and purification", Canadian Journal Of Biochemistry And Physiology vol. 37, pp.911-917, 1959). The extracted lipid fraction can be fractionated, e.g. by high-performance liquid chromatography (HPLC), and each lipid fraction can be analyzed and characterized, e.g. using HPLC coupled to a mass spectrometer (HPLC-MS), thereby identifying the presence or absence of PtdThr. Further, the amount of PtdThr and other lipids can be quantified by, e.g. performing two dimensional thin layer chromatography (2D-TLC) and a phosphorous analysis of the spots on the 2D-TLC as described by Rouser et al. (Two dimensional thin layer chromatographic separation of polar lipids and determination of phospholipids by phosphorus analysis of spots, Lipids, vol.5, pp. 494-496, 1970).

In several embodiments, disrupted coccidian parasites as described herein produce no PtdThr, or an amount of PtdThr, which is non-detectable by HPLC or 2D-TLC phosphorous analysis.

PTS-disrupted coccidian parasites as described herein are suitable for use as a vaccine. They may be used to vaccinate an animal or a human by administrating a coccidian parasite to said animal or human (e.g. by intra-peritoneal injection). Parasites as described herein may be preferably used to vaccinate an animal selected from sheep, pig, poultry and cattle. *T. gondii* may be a parasite of choice for sheep and pig vaccination in particular, *E. tenella* may be a parasite of choice for poultry vaccination in particular, and *N*. *caninum* may be a parasite of choice for cattle vaccination in particular. Of note is the fact that no chemotherapy and human vaccine are available to treat chronic toxoplasmosis. Therefore, prevention of zoonotic transmission by vaccinating the farm and food animals might be the best way forward to reduce and/or eradicate toxoplasmosis burden in human populations.

Also described is a polynucleotide comprising a nucleotide sequence encoding a phosphatidylthreonine synthase (PTS) enzyme.

A PTS-encoding polynucleotide may originate from, e.g. a coccidian parasite selected from *Toxoplasma, Neospora* and *Eimeria species,* or from a coccidian parasite selected from *Toxoplasma gondii, Neospora caninum* and *Eimeria tenella.* The coccidian parasite may preferably be *Toxoplasma gondii.*

Coccidian parasites, for instance, can be grown in a suitable medium to isolate the PTS-encoding polynucleotide, which can be cloned in a suitable vector for heterologous expression by methods known in the art. Suitable vectors include, for example, pQE60 for expression in Escherichia coli or pESC-Ura in Saccharomyces cerevisiae, which can be used for subsequent expression of PTS. As a mode of example pQE60 vector for overexpressing *Tg*PTS in *E. coli* may be used.

A polynucleotide as described herein may also be produced synthetically by methods known in the art.

A polynucleotide as described herein may comprise a nucleotide sequence encoding a protein having an amino acid sequence of SEQ ID No. 2 for *Tg*PTS, SEQ ID No. 4 for *Nc*PTS or SEQ ID No. 6 for *Et*PTS, or homologues, which encode an amino acid sequence displaying at least 30% identity to the indicated sequences, in particular at least 50%, more in particular at least 75%, even more in particular at least 90 %, yet more in particular at least 95 % and more in particular at least 99% identity.

Such homologous sequences may encompass polymorphisms that may exist in cells from different populations or within a population due to natural or intra-strain variation. A homologue may further be derived from species other than the species where the specified DNA or amino acid sequence originates from, or may be artificially designed and synthesized. The PTS proteins designated as SEQ ID Nos. 2, 4 and 6 are encoded by the PTS polynucleotides of *Toxoplasma gondii* (SEQ ID No. 1), *Neospora canium* (SEQ ID No. 3) and *Eimeria tenella* (SEQ ID No. 5), respectively.

The PTS activity of a protein encoded by, e.g. *T*. *gondii* PTS gene or homologous sequences, can be demonstrated by overexpression of the gene in a suitable host (e.g. in a *E. coli* or yeast or mammalian cells) and subsequent identification of PtdThr by, e.g., isolation of the lipid fraction of the organism and its analysis to identify the presence of PtdThrby, e.g., thin layer chromatography (TLC) or high-performance liquid chromatography (HPLC) and mass spectrometry, or using other methods known in the art. For instance, overexpression of PTS can be performed by cloning in the pQE60 vector followed by transformation of *E. coli.*

A polynucleotide comprising a nucleotide sequence encoding a phosphatidylthreonine synthase (PTS) may be useful in the development of diagnostic methods and diagnostic kits or in the development of vaccines and drugs. For instance, a polynucleotide sequence as described herein may serve to express PTS, which in turn may be used for the production of PtdThr. For example, detection of PtdThr in samples from the infected patients or animals can be used to diagnose parasitic infections. Further, the availability of PTS and PtdThr can assist in the development of targeted drugs by, e.g. modification of the enzyme substrate threonine or of the product PtdThr with inhibitory properties and assessment PTS activity in the presence of the modified chemicals.

Also described is a phosphatidylthreonine synthase (PTS), encoded by the nucleotide sequences described above, and to a phosphatidylthreonine (PtdThr) lipid as also described above.

The instant invention is further illustrated by the following examples without being limited thereto or thereby.

### Reference Example 1 - Identification and Characterization of PtdThr

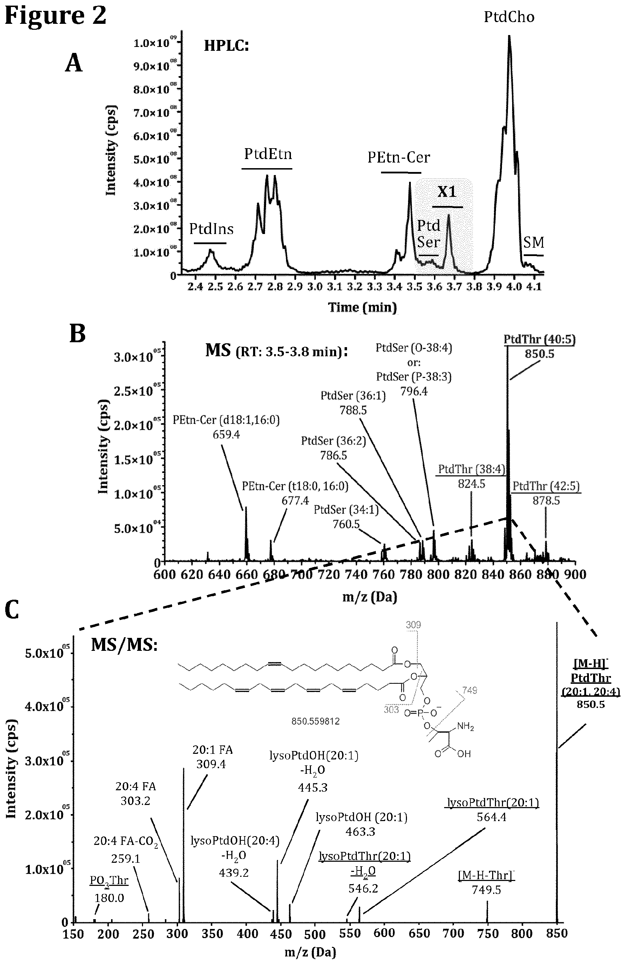

PtdThr was identified by HPLC fractionation of the lipids produced in *T. gondii* parasites as a major lipid peak. The acyl chain composition of PtdThr was determined by lipidomic analyses of the HPLC-derived fraction using mass spectrometry as described in detail below. The results are shown in Figure 2. The structure of a PtdThr is shown in Figure 2(C).

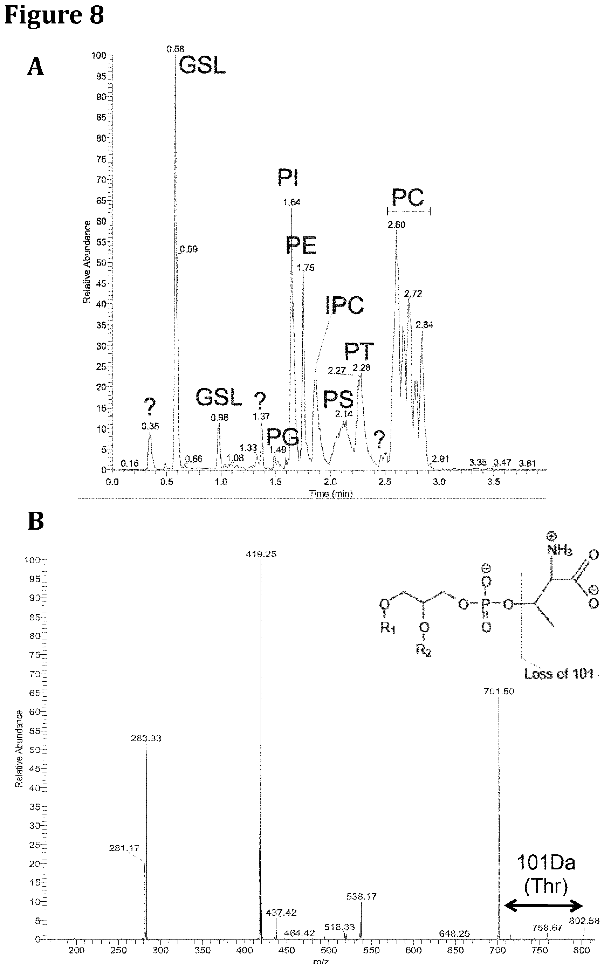

In a similar way PtdTHr was identified in *E. tenella* using chromatography and mass spectrometry (Figure 8 (A) and (B), wherein PtdThr is referred to as PT in Figure 8(A)).

### Parasite and host cell cultures

A Δku80 (type I) strain of *T. gondii* was provided by Vern B. Carruthers (University of Michigan, USA) (Huynh MH, Carruthers VB, agging of endogenous genes in a Toxoplasma gondii strain lacking Ku80, Eukaryot Cell. 2009 Apr;8(4):530-9). Tachyzoites of the Δku80 strain of *T. gondii* were propagated in human foreskin fibroblast (HFF) cells (obtained from ATCC, American type culture collection) using Dulbecco's Modified Eagle Medium (DMEM) containing fetal bovine serum (10%), glutamine (2 mM), minimum essential medium (MEM) non-essential amino acids (100 µm glycine, alanine, asparagine, aspartic acid, glutamic acid, proline, serine), sodium pyruvate (1 mM), penicillin (100 U/ml) and streptomycin (100 µg/ml) in a humidified incubator (37°C, 5% CO₂). Parasites were routinely cultured at a multiplicity of infection (MOI) of 3 every 2-3 days unless stated otherwise. HFF were harvested by trypsinization, and grown to confluence in fresh flasks, dishes or plates for infection assays as per experimental requirements.

### Lipid extraction

Parasites were syringe-released from infected HFF (MOI, 3; 42-48 hours of infection) and passed twice through 23G and 27G needles. Host debris was removed by filtering the parasite suspension through a 5 µm filter (Merck Millipore, Germany). Cell pellets were re-suspended in 0.4 ml of PBS and lipids were extracted according to Bligh-Dyer (Bligh, E. G. & Dyer, W. J. "A rapid method of total lipid extraction and purification", Canadian Journal Of Biochemistry And Physiology vol. 37, pp.911-917, 1959). Briefly, 0.5 ml chloroform and 1 ml methanol were added to the samples, which were then vortexed, allowed to stand for 30 min and then centrifuged (2000g, 5 min). The supernatant was transferred to a glass tube followed by addition of chloroform and 0.9% KCl (1 ml each). Samples were mixed, centrifuged and the lower chloroform phase containing lipids was transferred to a conical glass tube. Samples were stored at -20°C in the airtight glass tubes flushed with nitrogen gas until further use.

### Lipidomics analyses

Total lipids were fractionated on chloroform-equilibrated silica 60 columns. Neutral lipids were eluted by acetone washing of the column. Phospholipids were purified by 5x washing with 1 column-volume of chloroform/methanol/water (1:9:1). Each lipid fraction was collected, dried under nitrogen stream at 30°C, and stored at -20°C for downstream assays. Lipidomics was performed using automated HPLC electrospray or atmospheric-pressure-ionization tandem mass spectrometry. Internal standard PtdCho (44:2) was mixed with extracted lipids to calibrate the recovery of major lipids. A 10-20 µl aliquot of phospholipid extract in chloroform and methanol (1:1) was introduced onto a HILIC column (Kinetex, 2.6 µm) at a flow rate of 1 ml/min to separate phospholipid classes. MS data were collected using either a 4000 QTRAP (AB Sciex, Concord) or a LTQ-XL (Thermo Scientific, Hampton) mass spectrometer. Data were processed using the proprietary software of the respective instrument manufacturers.

The main lipidomic results of *T. gondii* tachyzoites are shown in Figure 2. Figure 2 (A) shows the HPLC elution profile with the retention times and relative abundance of phospholipids isolated from extracellular parasites (10⁷). X1, eluting between 3.5-3.8 min, represents the lipid identified as PtdThr. Figure 2 (B) shows the MS analysis of the X1 fraction revealing PtdThr, PtdSer and phosphoethanolamine-ceramide (PEtn-Cer) species. Individual lipids were identified by their fragmentation patterns and m/z ratios in the negative ionization mode. Figure 2 (C) shows the MS/MS spectrum of the X1-derived peak (m/z 850) from Figure 2(B). Note the neutral loss of 101 Da (850.5-749.5 transition) corresponding to the loss of polar head group threonine. The acyl chains were identified by their masses. The acyl chain of position sn-1 was identified as acyl 20:1 and the acyl of position sn-2 was identified as acyl 20:4. Sn-1 and sn-2 refer to the first and second carbon of the glycerol backbone in a given phospholipid. The third carbon contains the polar head group, such as threonine in PtdThr.

### Reference Example 2 - Identification and characterization of PTS

The genetic origin of PtdThr was determined by searching the parasite database Toxoplasma genomics resource database (ToxoDB) for expression of a relevant enzyme based on the structural similarity of threonine with serine. Two putative PtdSer synthases were found and their complete open reading frames were subsequently cloned (ToxoDB reference numbers TGGT1_273540 and TGGT1_261480). They were found to encode for 614 and 540 amino acid residues respectively. By sequence alignments with mammalian enzymes they were tentatively identified as close homologs of base-exchange type PtdSer synthases (PSS). The results of the alignment are presented in Figure 1. Based on *in silico* analyses and the wet-lab results described herein, the two synthases were named *Tg*PSS (*Toxoplasma gondii* phosphatidylserine synthase) and *Tg*PTS (*Toxoplasma gondii* phosphatidylthreonine synthase). Unlike PSS occurring across the phyla, homologs of PTS could only be found in selected parasitic (*Neospora, Eimeria, Phytophtora*) and free-living (*Perkinsus*) chromalveolates organisms. Of note is the fact that distinct asparagine, histidine and cysteine residues are strictly conserved in all PSS orthologs including in *Tg*PSS, however not in *Tg*PTS, which contains substitutions to glutamate, tryptophan and serine at the equivalent positions (Figure 1).

### Molecular cloning of T. gondii PTS

Parasites were syringe-released from infected HFF cells as described above for Example 1. The parasite RNA was isolated using Trizol-based method (Invitrogen) and was subsequently reverse-transcribed into first-strand cDNA.

The open reading frames of *Tg*PSS and *Tg*PTS were amplified from first-strand cDNA using PfuUltra II Fusion polymerase (Agilent Technologies). The primers used for amplification of the open reading frames are listed on table 1 (SEQ ID Nos. 7-10). The open reading frames were cloned into a commercial pDrive vector (Qiagen, Germany) following the manufacturer's protocol, which allowed DNA sequencing of the amplicons.

**Table 1 - PCR Primers for annotation of TgPTS and TgPSS open reading frames**

| **SEQ ID Number** | **Primer Name** | **Nucleotide Sequence** |
|---|---|---|
| 7 | *Tg*PTS-ORF-F | 5'-ATGCAACTCCCTTCAAGA-3' |
| 8 | *Tg*PTS-ORF-R | 5'-TCACTGACTTCGTTCCATTTTCACG-3' |
| 9 | *Tg*PSS-ORF-F | 5'-ATGTGTCGGGGACCGCCGCT-3' |
| 10 | *Tg*PSS-ORF-R | 5'-TCACTCGTCTTTTTGGCCTTC-3' |

### Example 3 - Genetic ablation of PTS in T. gondii

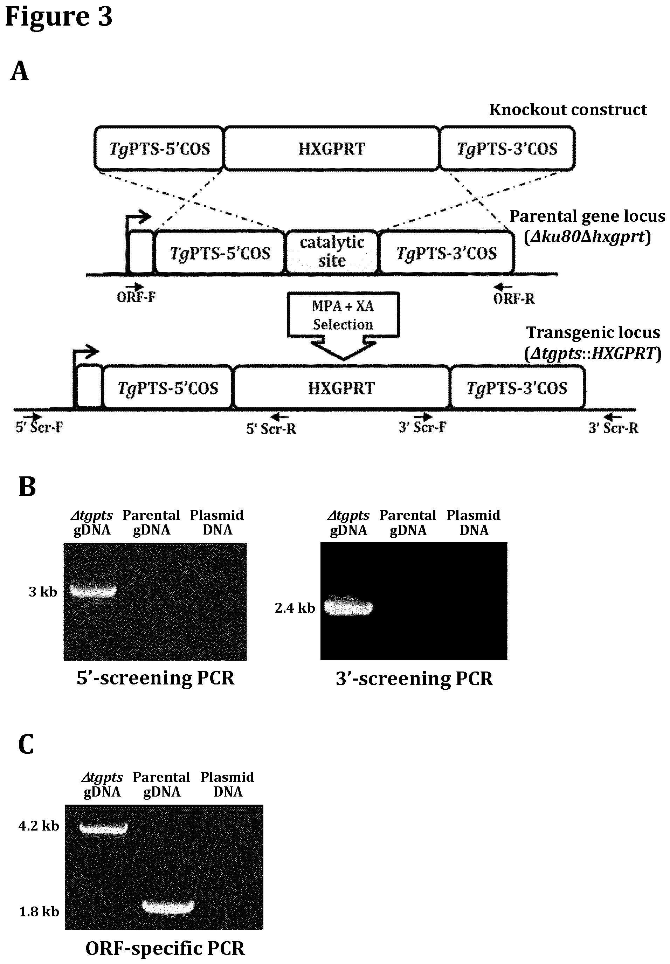

The PTS gene of *T. gondii* (*Tg*PTS) was disrupted by double homologous cross over. A knockout plasmid was constructed, which contained 5' and 3' crossover sequence (COS) of *Tg*PTS flanking a hypoxanthine xanthine guanine phosphoribosyltransferase (HXGPRT) marker as depicted in Figure 3 (A). The HXGPRT marker was used to allow transgenic selection by mycophenolic acid (MPA) and xanthine (XA) (Donald, et al. "Insertional tagging, cloning, and expression of the Toxoplasma gondii hypoxanthine-xanthine-guanine phosphoribosyltransferase gene. Use as a selectable marker for stable transformation", The Journal of Biological Chemistry, vol. 271, pp. 14010-14019, 1996).

### Knockout vector construction

The 5' and 3' crossover sequences (5'COS, 3'COS) of *Tg*PTS were amplified using the genomic DNA isolated from fresh extracellular tachyzoites and using the primers of Table 2 (SEQ ID Nos. 11-14).

The 5'COS (0.9 kb) and 3'COS (0.8 kb) were cloned at *Not*I/*EcoR*I and *Hpa*I/*Hpa*I sites of a *pTKO-HXGPRT* vector, respectively. The resulting knockout vector contained 5' and 3'COS of the *TgPTS* gene flanking a hypoxanthineguanine-phosphoribosyltransferase (HXGPRT) selection marker (*pTKO-5'COS-HXGPRT-3' COS) .*

**Table 2 - PCR Primers for cloning of the TgPTS-5'COS and the TgPTS-3'COS in the resulting TgPTS knockout vector**

| **SEQ ID Number** | **Primer Name (restriction site)** | **Nucleotide Sequence (restriction site underlined)** |
|---|---|---|
| 11 | *Tg*PTS-5'COS-F (*Not*I) | 5'-CTCATCGCGGCCGCGTTCGCCTCGAGTGCTTG-3' |
| 12 | *Tg*PTS-5'COS-R (*EcoR*I) | 5'-CTCATCGAATTCACGAGCCAGTGGAACGAC-3' |
| 13 | *Tg*PTS-3'COS-F (*Hpa*I) | 5'-CTCATCGTTAACAGCATCTTTATCGATGCGCT-3' |
| 14 | *Tg*PTS-3'COS-R (*Hpa*I) | 5'-CTCATCGTTAACTCACTGACTTCGTTCGATTTTC-3' |

### Genetic modification of T. gondii

The knockout plasmid constructs (also referred to as knockout vector) were transfected into fresh tachyzoites (*Δku80*) suspended in Cytomix (120 mM KCl, 0.15 mM CaCl₂, 10 mM K₂HPO₄/KH₂PO₄ pH 7.6, 25 mM HEPES pH 7.6, 2 mM EGTA, 56 mM MgCl₂) using a BTX instrument (*BTX Harvard Apparatus, USA*) with the following conditions: 50 µg DNA, about 10⁷ parasites, 2 kV, 50Ω, 25 µF and 250 µs. The knockout vector (*pTKO-5'COS-HXGPRT-3'COS*) permitted disruption of the *Tg*PTS gene to generate a *Δtgpts* mutant by mycophenolic acid (25 µg/ml) and xanthine (50 µg/ml) selection.

Single (clonal) drug-resistant mutant parasites were isolated by the limiting dilution method. The isolated PTS gene-disrupted parasites (i.e. lacking the conserved catalytic (ECWWD) site, also referred to as the Δtgpts mutant or Δtgpts strain), were screened for 5'- and 3'-crossover events at the *Tg*PTS gene locus by PCR, using screening primers 5'Scr-F/R and 3'Scr-F/R (Table 3, SEQ ID Nos. 15-18) pDrive vector (Qiagen, Germany) for standard cloning and DNA sequencing.

**Table 3 - PCR primers for 5' and 3' recombination screening of the Δtgpts mutant**

| **SEQ ID Number** | **Primer Name** | **Nucleotide Sequence** |
|---|---|---|
| 15 | *Tg*PTS-KO-5'Scr-F | 5'-CGATTCCTTGAGAGCAACTG-3' |
| 16 | *Tg*PTS-KO-5'Scr-R | 5'-GACGCAGATGTGCGTGTATC-3' |
| 17 | *Tg*PTS-KO-3'Scr-F | 5'-ACTGCCGTGTGGTAAAATGAA-3' |
| 18 | *Tg*PTS-KO-3'Scr-R | 5'-GCCATAGAGTTCATTGCGGACTC-3' |

The results of the screening are presented in Figure 3 (B), which displays PCR images of a representative clonal Δtgpts strain showing specific amplification of 3-kb and 2.4-kb long DNA bands by 5' and 3' screening. The parental gDNA and knockout plasmid were included as negative controls, respectively.

The *Δtgpts* mutant parasites were validated by the successful insertion of the selection marker (HXGPRT) at the *Tg*PTS locus by ORF-specific PCR using the primers *Tg*PTS-ORF-F and *Tg*PTS-ORF-R of Table 1 (SEQ ID Nos. 7-10).

The results are presented in Figure 3 (C), which displays the resulting ORF-specific PCR confirming a successful insertion of the selection marker in the PTS gene. The *Δtgpts* cDNA shows amplification of an expected 4.2-kb band as opposed to the 1.8-kb band in the parental cDNA, and none in the plasmid DNA, which corroborated the targeted insertion of selection marker and deletion of the predicted catalytic site (342ECWWD346).

The identity of all PCR amplicons was confirmed by sequencing, which was performed by the genomics company LGC Limited (Germany) using standard DNA sequencing methods.

### Example 4 - Effect of PTS ablation on PtdThr production in T. gondii

The phospholipid composition of the *Δtgpts* and parental strains was investigated by thin layer chromatography (TLC), lipid phosphorus assays and lipidomics analyses.

The total lipids were isolated from parasites as explained above for Example 1.

### Thin layer chromatography and phosphorus quantification

Lipids were resolved by two-dimensional TLC on silica 60 plates (Merck, Germany) using chloroform/methanol/ammonium hydroxide (65:35:5) and chloroform/acetic acid/methanol/water (75:25:5:2.2) as the solvents. They were visualized by staining with iodine vapors and/or ninhydrin spray, and were identified based on their co-migration with authentic standards (Avanti Lipids). The major iodine-stained phospholipid bands were scraped off the TLC plate, and quantified by phosphorus assay as described elsewhere (Rouser et al., "Two dimensional thin layer chromatographic separation of polar lipids and determination of phospholipids by phosphorus analysis of spots", Lipids, vol.5, pp. 494-496, 1970).

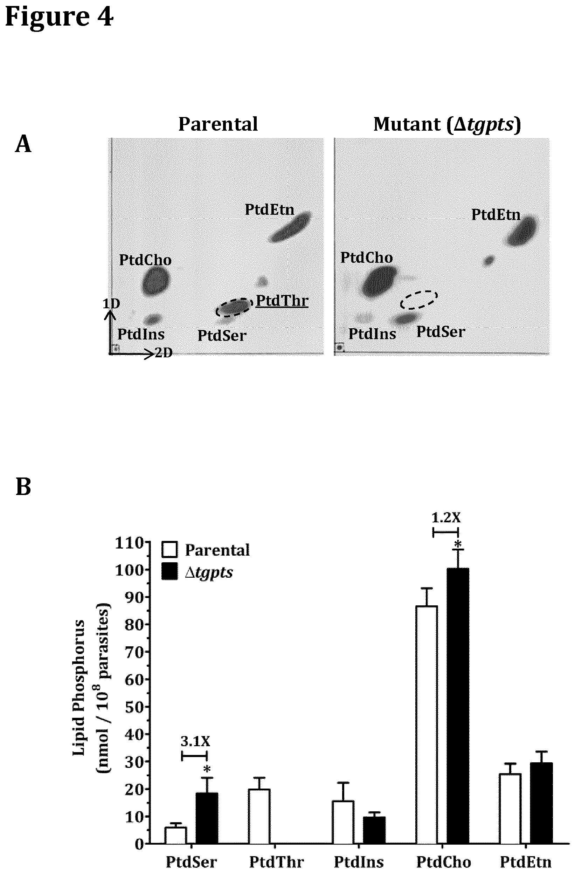

The results are presented in Figure 4.
Figure 4 (A) shows the lipid profiles of the indicated strains by two-dimensional TLC. The total lipids (0.8-1 x10⁸ parasites) were resolved and detected by iodine vapor staining. It is noted that the PdtThr band is absent in the *Δtgpts* mutant and that an increased production of PtdSer is also observed. Figure 4 (B) shows the lipid phosphorus measurements of bands from the TLC plates of Figure 4 (A) showing the lack of PtdThr and induction of PtdSer in the *Δtgpts* mutant. The reproducibility of the results was confirmed in four independent biological experiments (n=4).

As expected, the parental parasites harbored a notable amount of PtdThr (Figure 4A & B). Synthesis of PtdThr was completely abrogated in the *Δtgpts* strain. Concurrently, a three-fold increase in PtdSer was observed, whereas other lipids remained largely unaffected (Figure 4B). Genetic complementation of the mutant with a functional *Tg*PTS recovered PtdThr, as well as reversed PtdSer content to the normal level.

### Lipidomic analyses

The lipidomic analyses using HPLC/MS as described above for Example 1.

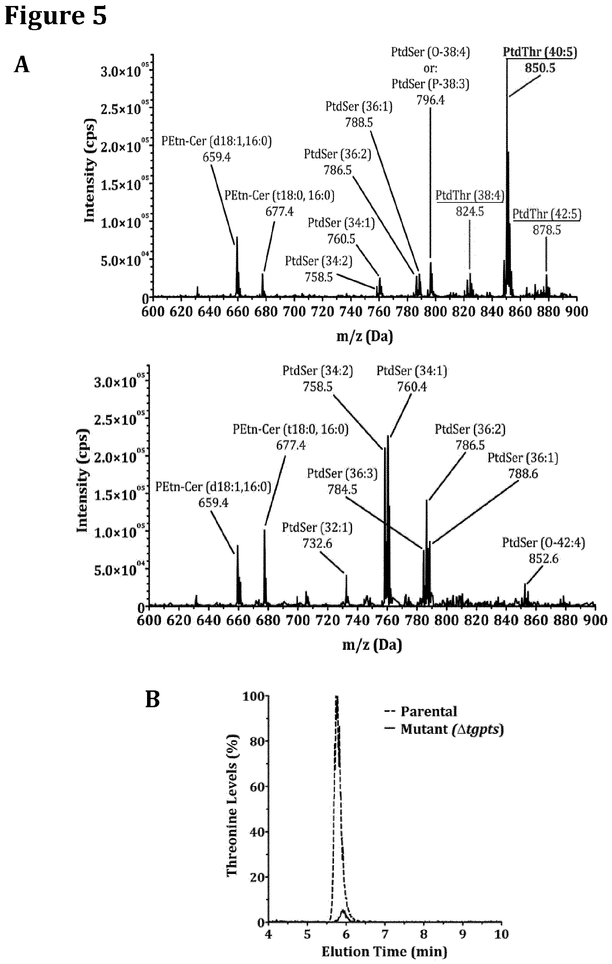

The results are presented in Figure 5. The lipidomics results confirmed the absence of all PtdThr-derived species in the mutant. In particular, Figure 5 (A) shows the representative MS profiles of the HPLC-resolved lipid (retention time, 3.5 - 3.8 min) from the parental and *Δtgpts* strains (∼107 parasites each) confirming the presence of a major (*m*/*z* 850.5, 40:5) and two minor (*m*/*z* 824.5, 38:4; *m*/*z* 878.5, 42:5) PtdThr species in the parental parasites, which are completely absent in the mutant. It is noted that PtdSer peaks are more intense in the *Δtgpts* sample, which is in agreement with lipid analysis by TLC and chemical phosphorus assays (Figure 4).

Taken together, these results show an autonomous synthesis of PtdThr by *Tg*PTS enzyme, and the loss of PtdThr lipid following its functional ablation in *T. gondii.*

### Example 5 - Effect of PTS ablation in T. gondii on gliding motility and virulence of T. gondii

### Gliding motility assays

Parasites (syringe-released from infected HFF as explained above) were incubated on BSA (0.01%)-coated coverslips in Hanks Balanced Salt Solution (HBSS) for 15 min at 37°C. Samples were fixed in 4% paraformaldehyde and 0.05% glutaraldehyde (10 min), and stained with anti-TgSagl primary antibody (donated by Jean-Franois Dubremetz, University of Montpellier, France) and Alexa488-conjugated secondary antibody (Life Technologies, Germany). The motile fraction and the trail length were quantified using the ImageJ software (National Institute of Health, USA).

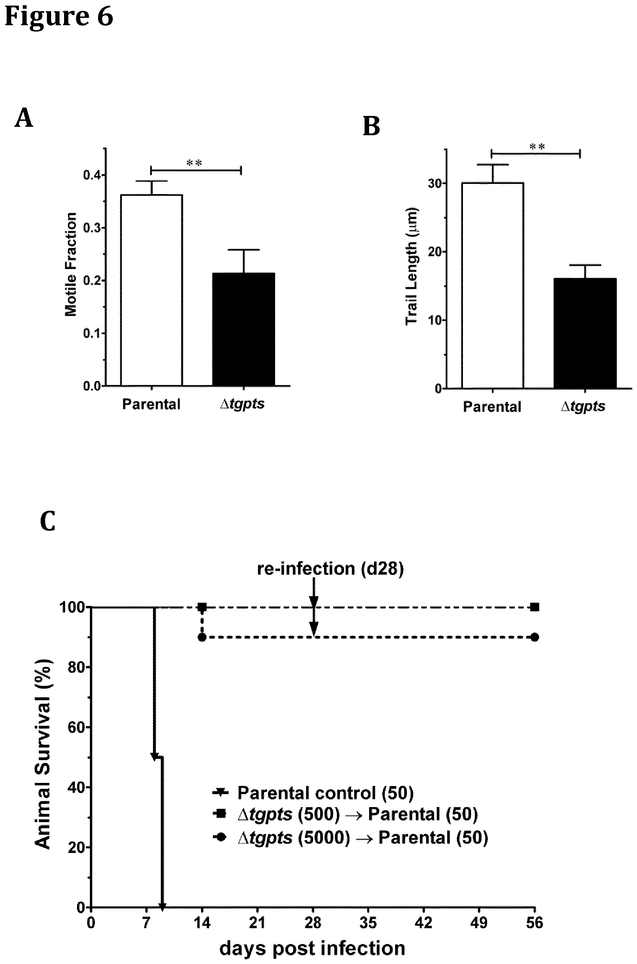

The results are shown in Figure 6 (A & B).
Figure 6 (A) shows the motile fractions of different strains on BSA-coated glass coverslips. In total, 500-1000 parasites from 6 independent assays were scored for the presence or absence of a motility trail (immuno-stained with anti-*Tg*Sagl antibody) to calculate the motile fractions for each strain. Figure 6 (B) shows the motility of individual parasite strains, as deduced by the length of *Tg*Sag1-stained trails. The error bars for Figure 6 (A & B) indicate the mean ± SEM. *p<0.05, **p<0.01, and ***p<0.001.

As depicted in Figure 6 (A & B), the *Δtgpts* strain showed a distinguished reduction in the motile fraction and the trail length when compared to the parental. The strain complemented with PTS activity showed a reverted phenotype similar to the parental strain, ascertaining the specificity of the observation. Such reduced parasite motility eventually causes a poor invasion and egress of/from HFF host cells by the *Δtgpts* mutant. A collective defect in motility, invasion and egress impairs the lytic cycle and virulence of the *Δtgpts* mutant.

### In vivo parasite infection for virulence testing

C57BL/6 mice (obtained from Janvier Labs, Saint Berthevin, France) were infected with tachyzoites of the *Δku80* (parental) or *Δtgpts* strains. Parasites for *in vivo* infections were propagated in HFF cells; fresh host-free tachyzoites were released 40 hrs post-infection and filtered (5 pm), as described above. They were injected *via* intra-peritoneal route (50 parasites of the parental strain and 5x10² or 5x10³ parasites of the *Δtgpts* strain). 12 animals were monitored for the mortality and morbidity 3 times a day over a period of 4 weeks. An inoculum of 50 parental tachyzoites was used to challenge the Δ*tgpts*-infected surviving animals, which were monitored for additional 4 weeks. A control group of naïve mice (n=4) was also infected with the same parental inoculum. The results are shown in Figure 6 (C).

### Quantification of T. gondii in the mouse brain

Cysts of the ME49 strain of *T. gondii* were harvested from the brains of female NMRI mice infected with *T. gondii* cysts 5 to 6 months earlier intraperitoneally as described elsewhere (Agrawal, G. G. van Dooren, W. L. Beatty, B. Striepen, Genetic evidence that an endosymbiont-derived endoplasmic reticulum-associated protein degradation (ERAD) system functions in import of apicoplast proteins, The Journal of Biological Chemistry 284, 33683-91 (2009)). The *Δtgpts*-vaccinated mice (500 parasites) were challenged with the ME49 *T*. *gondii strain* (3 cysts in 200 µl) parasites 4 weeks after vaccination. A control group of naive animals was also included. Parasite burden in the mouse brain was estimated by counting cysts and semi-quantitative real-time PCR following another 4 weeks of infection with the ME49 *T. gondii* strain. Brain tissue was mechanically homogenized in 1 ml sterile phosphate-buffered saline and the cysts were counted using a light microscope. For quantitative PCR (qPCR), perfused brain tissue samples were snap-frozen and stored at -80°C. 30 mg tissue was used to purify nucleic acids (QIAgen, Germany). FastStart Essential DNA Green Master (Roche, Germany) was mixed with genomic DNA (90 ng) in triplicate reactions, which were developed in a LightCycler® 480 Instrument II (Roche, Germany). The parasite burden (target: *Tg*B1 gene) was analyzed relative to mouse (reference: argininosuccinate lyase (*Mm*ASL)) by estimating target-to-reference ratio (LightCycler® 480 software v1.5.0). Primers used to amplify the *Tg*B1 and *Mm*ASL genes are listed in Table 4 (SEQ ID Nos. 19-22) .

**Table 4 - PCR primers for amplification of the TgB1 and MmASL genes**

| **SEQ ID Number** | **Primer Name** | **Nucleotide Sequence** |
|---|---|---|
| 19 | *Tg*B1-F | 5'-TCCCCTCTGCTGGCGAAAAGT-3' |
| 20 | *Tg*B1-R | 5'-AGCGTTCGTGGTCAACTATCGATTG-3' |
| 21 | *Mm*ASL-F | 5'-TCTTCGTTAGCTGGCAACTCACCT-3' |
| 22 | *Mm*ASL-R | 5'-ATGACCCAGCAGCTAAGCAGATCA-3' |

All in vivo assays were in compliance with the German animal protection laws directed by Landesverwaltungsamt Sachsen-Anhalt, Germany.

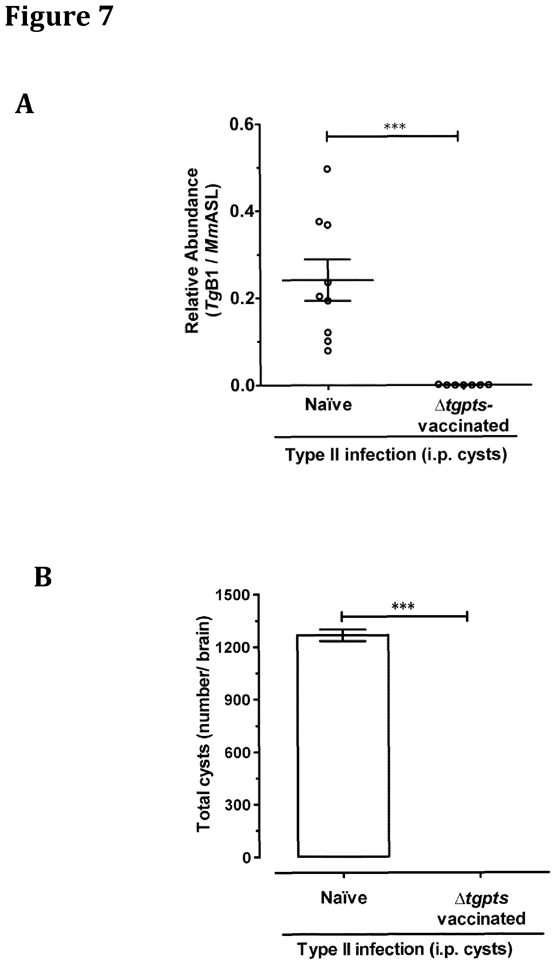

The results are shown in Figure 7.

Examination of virulence in a murine model demonstrated that nearly all animals infected with the *Δtgpts* mutant survived as opposed to the parental strain that was explicitly lethal (Figure 6C). Importantly, all mice enduring the mutant infection became categorically resistant to a subsequent lethal challenge by a hypervirulent *Δku80* strain of *T. gondii* (type I infection) causing acute toxoplasmosis (Figure 6C). To further expand the utility of our strain as a potential vaccine against chronic infection, the Δ*tgpts*-infected animals were challenged with the cyst-forming ME49 *T. gondii* strain (type II infection). Surprisingly, in contrast to naive animals, the mutant-vaccinated mice showed no signs of chronic stage cysts in their brain tissue (Figure 7A-B). Consistently, unlike the infected naive control mice, no inflammatory lesions in the cortex or meninges of the Δ*tgpts*-immunized animals were observed. In brief, these results demonstrate the *in vivo* requirement of PtdThr for the parasite virulence, and illustrate the prophylactic potential of a metabolically attenuated whole-cell 'vaccine' against acute as well as chronic toxoplasmosis.

## Claims

1. A coccidian parasite selected from *Toxoplasma, Neospora* and *Eimeria* species wherein the expression of endogenous phosphatidylthreonine synthase (PTS) enzyme is disrupted by genetic modification thereby eliminating the synthesis of a phosphatidylthreonine (PtdThr).

2. The coccidian parasite of claim 1 which is selected from *Toxoplasma gondii, Neospora caninum* and *Eimeria tenella,* or is *Toxoplasma gondii.*

3. The coccidian parasite of claim 1 or 2 wherein the PtdThr is a PtdThr of formula (I): 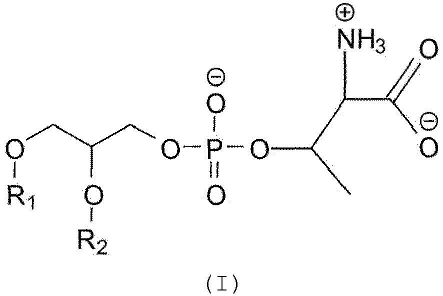 wherein R₁ and R₂ are independently selected from saturated and/or unsaturated acyl groups having from 8 to 46 carbon atoms.

4. The coccidian parasite of any one of claims 1 to 3, wherein the expression of PTS enzyme is disrupted by inactivating or deleting the corresponding PTS-encoding gene.

5. The coccidian parasite of any one of claims 1 to 4 wherein the expression of PTS enzyme is disrupted by inactivating or deleting a nucleotide sequence encoding a protein sequence with at least 30% identity to *Toxoplasma gondii PTS* (SEQ ID No. 2), *Neospora caninum* PTS (SEQ ID No. 4) or *Eimeria tenella* PTS (SEQ ID No. 6), in particular at least 50%, more in particular at least 75%, even more in particular at least 90 %, yet more in particular at least 95 % and more in particular at least 99%.

6. The coccidian parasite of any one of claims 1 to 5, wherein the expression of PTS enzyme is disrupted by deleting or replacing the polynucleotide sequence, which comprises the nucleotides encoding for the catalytic site of PTS.

7. The coccidian parasite of any one of claims 1 to 6 for use as a vaccine.

8. A method for preparing a genetically modified coccidian parasite of any one of claims 1 to 7, which comprises disrupting the expression of endogenous phosphatidylthreonine synthase (PTS) enzyme in a coccidian parasite selected from *Toxoplasma, Neospora* and *Eimeria* species by inactivating or deleting the gene encoding PTS thereby eliminating the synthesis of phosphatidylthreonine (PtdThr).

9. The method of claim 8, which comprises inactivating or deleting the gene encoding PTS enzyme by single or double homologous recombination.

10. The coccidian parasite of any one of claims 1 to 6 for use in the prevention of coccidian parasite-inflicted diseases caused by Toxoplasma, Neospora, and Eimeria species in animals or humans.

11. The coccidian parasite for the use of claim 10, wherein the animal is selected from sheep, pig, poultry and cattle.

12. A vaccine comprising a genetically modified coccidian parasite according to any one of claims 1 to 6.

## Patentansprüche

1. Kokzidienparasit ausgewählt aus *Toxoplasma-, Neospora-* und *Eimeria-Arten,* wobei die Expression des endogenen Phosphatidylthreoninsynthase (PTS)-Enzyms durch genetische Modifikation gestört ist, wodurch die Synthese eines Phosphatidylthreonins (PtdThr) ausgeschaltet ist.

2. Kokzidienparasit nach Anspruch 1, der aus *Toxoplasma gondii, Neospora caninum* und *Eimeria tenella* ausgewählt ist oder *Toxoplasma gondii* ist.

3. Kokzidienparasit nach Anspruch 1 oder 2, wobei das PtdThr ein PtdThr der Formel (I) 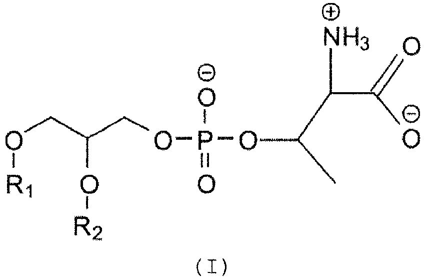 ist,
wobei R₁ und R₂ unabhängig voneinander ausgewählt sind aus gesättigten und/oder ungesättigten Acylgruppen mit 8 bis 46 Kohlenstoffatomen.

4. Kokzidienparasit nach einem der Ansprüche 1 bis 3, wobei die Expression des PTS-Enzyms durch Inaktivieren oder Deletieren des entsprechenden PTS-codierenden Gens gestört ist.

5. Kokzidienparasit nach einem der Ansprüche 1 bis 4, wobei die Expression des PTS-Enzyms durch Inaktivieren oder Deletieren einer Nucleotidsequenz gestört ist, die eine Proteinsequenz mit mindestens 30% Identität mit *Toxoplasma gondii-PTS* (SEQ ID NO:2), *Neospora caninum-PTS* (SEQ ID NO:4) oder *Eimeria tenella*-PTS (SEQ ID NO:6) codiert, bevorzugt mindestens 50%, stärker bevorzugt mindestens 75%, noch stärker bevorzugt 90% und sogar noch stärker bevorzugt mindestens 95% und stärker bevorzugt mindestens 99%.

6. Kokzidienparasit nach einem der Ansprüche 1 bis 5, wobei die Expression des PTS-Enzyms durch Deletieren oder Ersetzen der Polynucleotidsequenz, die die Nucleotide umfasst, die die katalytische Stelle von PTS codieren, gestört ist.

7. Kokzidienparasit nach einem der Ansprüche 1 bis 6 zur Verwendung als ein Impfstoff.

8. Verfahren zur Herstellung eines genetisch modifizierten Kokzidienparasiten nach einem der Ansprüche 1 bis 7, das das Stören der Expression des endogenen Phosphatidylthreoninsynthase (PTS)-Enzyms in einem Kokzidienparasiten ausgewählt aus *Toxoplasma-, Neospora-* und *Eimeria-*Arten durch Inaktivieren oder Deletieren des PTS-codierenden Gens umfasst, wodurch die Synthese von Phosphatidylthereonin (PtdThr) ausgeschaltet wird.

9. Verfahren nach Anspruch 8, das das Inaktivieren oder Deletieren des Gens, das das PTS-Enzym codiert, durch einfache oder doppelte homologe Rekombination umfasst.

10. Kokzidienparasit nach einem der Ansprüche 1 bis 6 zur Verwendung bei der Vorbeugung von durch Kokzidienparasiten hervorgerufenen Erkrankungen, die von *Toxoplasma-, Neospora-* und *Eimeria-Arten* bei Tieren oder Menschen verursacht sind.

11. Kokzidienparasit zur Verwendung nach Anspruch 10, wobei das Tier aus Schaf, Schwein, Geflügel und Rind ausgewählt ist.

12. Impfstoff, umfassend einen genetisch modifizierten Kokzidienparasiten nach einem der Ansprüche 1 bis 6.

## Revendications

1. Parasite coccidien choisi parmi les espèces *Toxoplasma, Neospora,* et *Eimeria,* dans lequel l'expression de l'enzyme phosphatidylthréonine synthase (PTS) endogène est interrompue par modification génétique, éliminant ainsi la synthèse d'une phosphatidylthréonine (PtdThr).

2. Parasite coccidien selon la revendication 1, qui est choisi parmi *Toxoplasma gondii, Neospora caninum* et *Eimeria tenella,* ou est *Toxoplasma gondii.*

3. Parasite coccidien selon la revendication 1 ou 2, dans lequel la PtdThr est une PtdThr de formule (I) : 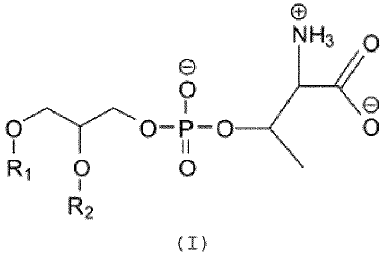 dans laquelle R₁ et R₂ sont indépendamment choisis parmi des groupes acyle saturés et/ou insaturés comportant de 8 à 46 atomes de carbone.

4. Parasite coccidien selon l'une quelconque des revendications 1 à 3, dans lequel l'expression de l'enzyme PTS est interrompue par inactivation ou délétion du gène correspondant codant PTS.

5. Parasite coccidien selon l'une quelconque des revendications 1 à 4, dans lequel l'expression de l'enzyme PTS est interrompue par inactivation ou délétion d'une séquence de nucléotides codant une séquence de protéine avec au moins 30 % d'identité avec PTS de *Toxoplasma gondii* (SEQ ID N° 2), PTS de *Neospora caninum* (SEQ ID N° 4) ou PTS d'*Eimeria tenella* (SEQ ID N° 6), en particulier au moins 50 %, plus particulièrement au moins 75 %, encore plus particulièrement au moins 90 %, toujours plus particulièrement au moins 95 % et tout particulièrement au moins 99 %.

6. Parasite coccidien selon l'une quelconque des revendications 1 à 5, dans lequel l'expression de l'enzyme PTS est interrompue par délétion ou remplacement de la séquence de nucléotides, qui comprend les nucléotides codant le site catalytique de PTS.

7. Parasite coccidien selon l'une quelconque des revendications 1 à 6 à utiliser comme vaccin.

8. Procédé de préparation d'un parasite coccidien génétiquement modifié selon l'une quelconque des revendications 1 à 7, qui comprend l'interruption de l'expression de l'enzyme phosphatidylthréonine synthase (PTS) endogène dans un parasite coccidien choisi parmi les espèces *Toxoplasma, Neospora,* et *Eimeria* par inactivation ou délétion du gène codant PTS éliminant ainsi la synthèse de phosphatidylthréonine (PtdThr).

9. Procédé selon la revendication 8, qui comprend l'inactivation ou la délétion du gène codant l'enzyme PTS par recombinaison homologue simple ou double.

10. Parasite coccidien selon l'une quelconque des revendications 1 à 6, à utiliser dans la prévention de maladies infligées par parasite coccidien provoquées par les espèces *Toxoplasma, Neospora,* et *Eimeria* chez des animaux ou humains.

11. Parasite coccidien à utiliser selon la revendication 10, dans lequel l'animal est choisi parmi un mouton, un cochon, de la volaille et du bétail.

12. Vaccin comprenant un parasite coccidien génétiquement modifié selon l'une quelconque des revendications 1 à 6.


### SEQUENCE LISTING

<110> Humboldt University & Utrecht University
<120> Genetically modified coccidian parasites useful as vaccines
<130> 20864
<160> 40
<170> PatentIn version 3.5
<210> 1
   <211> 1845
   <212> DNA
   <213> Toxoplasma gondii
<220>
   <221> CDS
   <222> (1)..(1845)
<400> 1
   atg caa ctc cct tca aga aag gag tcc gga acc ccc aac aat gcg gcg      48
   Met Gln Leu Pro Ser Arg Lys Glu Ser Gly Thr Pro Asn Asn Ala Ala
   1               5                   10                  15
   tca cca ggc agc cta aag tca gcg aga acc aca cgc gct tcg agt ccc      96
   Ser Pro Gly Ser Leu Lys Ser Ala Arg Thr Thr Arg Ala Ser Ser Pro
               20                  25                  30
   tcg atg gcc gag tgt tcg cct cga gtg ctt gtc aac tgc ggt gtc gct     144
   Ser Met Ala Glu Cys Ser Pro Arg Val Leu Val Asn Cys Gly Val Ala
           35                  40                  45
   ccg act gag caa ggg gcc cat ggt gag cat aaa agc ttg gaa gtc tcc     192
   Pro Thr Glu Gln Gly Ala His Gly Glu His Lys Ser Leu Glu Val Ser
       50                  55                  60
   act gac aac tca gct tgc aag gaa ccc cgg cgc atg ctc tgg ggt tca     240
   Thr Asp Asn Ser Ala Cys Lys Glu Pro Arg Arg Met Leu Trp Gly Ser
   65                  70                  75                  80
   att ctc atc ctg agt tta ctg ctt ctc ggg ttc tgg gac cag ctg ttt     288
   Ile Leu Ile Leu Ser Leu Leu Leu Leu Gly Phe Trp Asp Gln Leu Phe
                   85                  90                  95
   ccg cct tcc cag atg aaa gat gtc gtg cgc tct gtc cac gaa ggt gcc     336
   Pro Pro Ser Gln Met Lys Asp Val Val Arg Ser Val His Glu Gly Ala
               100                 105                 110
   ggg aaa cct ggc gat gtt gct ggt tct gca gag ctt cct gca gaa agg     384
   Gly Lys Pro Gly Asp Val Ala Gly Ser Ala Glu Leu Pro Ala Glu Arg
           115                 120                 125
   tgt gtc gag gga gag tgg aag agg gac aag ggg gga gag tct gca cca     432
   Cys Val Glu Gly Glu Trp Lys Arg Asp Lys Gly Gly Glu Ser Ala Pro
       130                 135                 140
   ggt gac gac ccc gcg tgc caa aaa ggc gcc aca caa gcg acg cct cgt     480
   Gly Asp Asp Pro Ala Cys Gln Lys Gly Ala Thr Gln Ala Thr Pro Arg
   145                 150                 155                 160
   cca tct gag gaa cac gcg gga gcg gcg aaa cca acc cct gca act cct        528
   Pro Ser Glu Glu His Ala Gly Ala Ala Lys Pro Thr Pro Ala Thr Pro
                   165                 170                 175
   tcc gtg ttt tct tca ctc ctt ggt gat tgg tca tct ccc gtt tcc agc        576
   Ser Val Phe Ser Ser Leu Leu Gly Asp Trp Ser Ser Pro Val Ser Ser
               180                 185                 190
   tct cca aag agc gcg aat gtc agg cgt gtc gtg cta gcg ctt ctc att        624
   Ser Pro Lys Ser Ala Asn Val Arg Arg Val Val Leu Ala Leu Leu Ile
           195                 200                 205
   ctt gtc tgc atg tac tgt ttc atc cag gcg aga gac tgc tca ctc gtt        672
   Leu Val Cys Met Tyr Cys Phe Ile Gln Ala Arg Asp Cys Ser Leu Val
       210                 215                 220
   ctc ccg cac ccc ggc ttc tgg cgt gtg gtg cac ggc atg tgc gtc gtt        720
   Leu Pro His Pro Gly Phe Trp Arg Val Val His Gly Met Cys Val Val
   225                 230                 235                 240
   cac ttg att gtc atg gtg gtg ctg ctg gtc gtt gac gaa gag acg gga        768
   His Leu Ile Val Met Val Val Leu Leu Val Val Asp Glu Glu Thr Gly
                   245                 250                 255
   aga cac gcg ttg gag ttg ctg ttc ccc gaa atc gca gga aaa aga gag        816
   Arg His Ala Leu Glu Leu Leu Phe Pro Glu Ile Ala Gly Lys Arg Glu
               260                 265                 270
   gaa att ttt gcg ggg aca cta gtg gta gac tgc cga atc aat gca agc        864
   Glu Ile Phe Ala Gly Thr Leu Val Val Asp Cys Arg Ile Asn Ala Ser
           275                 280                 285
   acg atc cac cga cag ttg aca agt gtg tgg ttc gtc tcc cac gtt gtt        912
   Thr Ile His Arg Gln Leu Thr Ser Val Trp Phe Val Ser His Val Val
       290                 295                 300
   ggc tgg ctt gta aag atg gtc att ctc cga cac tgg ggg ttt tgt ctc        960
   Gly Trp Leu Val Lys Met Val Ile Leu Arg His Trp Gly Phe Cys Leu
   305                 310                 315                 320
   atc tac tcc ctt tgc ttt gaa cta ggc gaa ctg tcg ttc cac tgg ctc       1008
   Ile Tyr Ser Leu Cys Phe Glu Leu Gly Glu Leu Ser Phe His Trp Leu
                   325                 330                 335
   gtc ccc gaa ctc tgc gaa tgc tgg tgg gac agc atc ttt atc gat gcg       1056
   Val Pro Glu Leu Cys Glu Cys Trp Trp Asp Ser Ile Phe Ile Asp Ala
               340                 345                 350
   ctg ctc agc aac gtt tgc ggc atg ttt ctg ggg gtc ctc ttc atg aag       1104
   Leu Leu Ser Asn Val Cys Gly Met Phe Leu Gly Val Leu Phe Met Lys
           355                 360                 365
   ctt gtc aac ctg tgc cag tac gac tgg cta ggc cgc tac ccg cag gat       1152
   Leu Val Asn Leu Cys Gln Tyr Asp Trp Leu Gly Arg Tyr Pro Gln Asp
       370                 375                 380
   cag aac gtt tcc att agt ctg act ccg ttt gca tct gaa gct tcc gac       1200
   Gln Asn Val Ser Ile Ser Leu Thr Pro Phe Ala Ser Glu Ala Ser Asp
   385                 390                 395                 400
   tgg agc ttc tat aaa acg ccc cgc cat ctg ttc atg tct atc gtg ctc       1248
   Trp Ser Phe Tyr Lys Thr Pro Arg His Leu Phe Met Ser Ile Val Leu
                   405                 410                 415
   ctt atg atc tgt ctc ttt tcg gag ctg aac gtc ttc ttt ctt atg gca        1296
   Leu Met Ile Cys Leu Phe Ser Glu Leu Asn Val Phe Phe Leu Met Ala
               420                 425                 430
   gcc ctt gac atc cct gct gcg cac tac agc aac cct ctc cgt acc ttg        1344
   Ala Leu Asp Ile Pro Ala Ala His Tyr Ser Asn Pro Leu Arg Thr Leu
           435                 440                 445
   tat atc gct ctc cta gga gtt gcc gca gct gca gag cac tat gaa ttc        1392
   Tyr Ile Ala Leu Leu Gly Val Ala Ala Ala Ala Glu His Tyr Glu Phe
       450                 455                 460
   acc acg aac cgc cgc gac aga att ggc cgg aat aag tgg tta ctc att        1440
   Thr Thr Asn Arg Arg Asp Arg Ile Gly Arg Asn Lys Trp Leu Leu Ile
   465                 470                 475                 480
   atc att ctc ttt gtc gaa tgg ctg gtg tgc gca aaa tac gga gcg cgc        1488
   Ile Ile Leu Phe Val Glu Trp Leu Val Cys Ala Lys Tyr Gly Ala Arg
                   485                 490                 495
   cgg tac gct gca ggg ctc cct ccc ccc gac att ttt gtg ccg tgg att        1536
   Arg Tyr Ala Ala Gly Leu Pro Pro Pro Asp Ile Phe Val Pro Trp Ile
               500                 505                 510
   gtt agc tcc atc ctc ttt tct ctg tgg tgt tac tgt tac tat acc aca        1584
   Val Ser Ser Ile Leu Phe Ser Leu Trp Cys Tyr Cys Tyr Tyr Thr Thr
           515                 520                 525
   gct gac ctc cgc aca gac agt ggg aaa ggg aag ctg ctg gcg aag tct        1632
   Ala Asp Leu Arg Thr Asp Ser Gly Lys Gly Lys Leu Leu Ala Lys Ser
       530                 535                 540
   ccc ggc ggg aaa gga gcg ggt gac gag agc gca aat ctg gca gca gag        1680
   Pro Gly Gly Lys Gly Ala Gly Asp Glu Ser Ala Asn Leu Ala Ala Glu
   545                 550                 555                 560
   tcg aaa atg aca aag cgg tca gct aag cag tgt cca gcg gct tcc tgg        1728
   Ser Lys Met Thr Lys Arg Ser Ala Lys Gln Cys Pro Ala Ala Ser Trp
                   565                 570                 575
   cag gat cgg atc aag ctg ctc gtt cta aca tta ccc cct cag gca tgc        1776
   Gln Asp Arg Ile Lys Leu Leu Val Leu Thr Leu Pro Pro Gln Ala Cys
               580                 585                 590
   ttt atg ccg ctg ctg tat ctc tcg aaa ttc tac ttt ttc gac tac gtg        1824
   Phe Met Pro Leu Leu Tyr Leu Ser Lys Phe Tyr Phe Phe Asp Tyr Val
           595                 600                 605
   aaa atc gaa cga agt cag tga                                            1845
   Lys Ile Glu Arg Ser Gln
       610
<210> 2
   <211> 614
   <212> PRT
   <213> Toxoplasma gondii
<400> 2
   Met Gln Leu Pro Ser Arg Lys Glu Ser Gly Thr Pro Asn Asn Ala Ala
   1               5                   10                  15
   Ser Pro Gly Ser Leu Lys Ser Ala Arg Thr Thr Arg Ala Ser Ser Pro
               20                  25                  30
   Ser Met Ala Glu Cys Ser Pro Arg Val Leu Val Asn Cys Gly Val Ala
           35                  40                  45
   Pro Thr Glu Gln Gly Ala His Gly Glu His Lys Ser Leu Glu Val Ser
       50                  55                  60
   Thr Asp Asn Ser Ala Cys Lys Glu Pro Arg Arg Met Leu Trp Gly Ser
   65                  70                  75                  80
   Ile Leu Ile Leu Ser Leu Leu Leu Leu Gly Phe Trp Asp Gln Leu Phe
                   85                  90                  95
   Pro Pro Ser Gln Met Lys Asp Val Val Arg Ser Val His Glu Gly Ala
               100                 105                 110
   Gly Lys Pro Gly Asp Val Ala Gly Ser Ala Glu Leu Pro Ala Glu Arg
           115                 120                 125
   Cys Val Glu Gly Glu Trp Lys Arg Asp Lys Gly Gly Glu Ser Ala Pro
       130                 135                 140
   Gly Asp Asp Pro Ala Cys Gln Lys Gly Ala Thr Gln Ala Thr Pro Arg
   145                 150                 155                 160
   Pro Ser Glu Glu His Ala Gly Ala Ala Lys Pro Thr Pro Ala Thr Pro
                   165                 170                 175
   Ser Val Phe Ser Ser Leu Leu Gly Asp Trp Ser Ser Pro Val Ser Ser
               180                 185                 190
   Ser Pro Lys Ser Ala Asn Val Arg Arg Val Val Leu Ala Leu Leu Ile
           195                 200                 205
   Leu Val Cys Met Tyr Cys Phe Ile Gln Ala Arg Asp Cys Ser Leu Val
       210                 215                 220
   Leu Pro His Pro Gly Phe Trp Arg Val Val His Gly Met Cys Val Val
   225                 230                 235                 240
   His Leu Ile Val Met Val Val Leu Leu Val Val Asp Glu Glu Thr Gly
                   245                 250                 255
   Arg His Ala Leu Glu Leu Leu Phe Pro Glu Ile Ala Gly Lys Arg Glu
               260                 265                 270
   Glu Ile Phe Ala Gly Thr Leu Val Val Asp Cys Arg Ile Asn Ala Ser
           275                 280                 285
   Thr Ile His Arg Gln Leu Thr Ser Val Trp Phe Val Ser His Val Val
       290                 295                 300
   Gly Trp Leu Val Lys Met Val Ile Leu Arg His Trp Gly Phe Cys Leu
   305                 310                 315                 320
   Ile Tyr Ser Leu Cys Phe Glu Leu Gly Glu Leu Ser Phe His Trp Leu
                   325                 330                 335
   Val Pro Glu Leu Cys Glu Cys Trp Trp Asp Ser Ile Phe Ile Asp Ala
               340                 345                 350
   Leu Leu Ser Asn Val Cys Gly Met Phe Leu Gly Val Leu Phe Met Lys
           355                 360                 365
   Leu Val Asn Leu Cys Gln Tyr Asp Trp Leu Gly Arg Tyr Pro Gln Asp
       370                 375                 380
   Gln Asn Val Ser Ile Ser Leu Thr Pro Phe Ala Ser Glu Ala Ser Asp
   385                 390                 395                 400
   Trp Ser Phe Tyr Lys Thr Pro Arg His Leu Phe Met Ser Ile Val Leu
                   405                 410                 415
   Leu Met Ile Cys Leu Phe Ser Glu Leu Asn Val Phe Phe Leu Met Ala
               420                 425                 430
   Ala Leu Asp Ile Pro Ala Ala His Tyr Ser Asn Pro Leu Arg Thr Leu
           435                 440                 445
   Tyr Ile Ala Leu Leu Gly Val Ala Ala Ala Ala Glu His Tyr Glu Phe
       450                 455                 460
   Thr Thr Asn Arg Arg Asp Arg Ile Gly Arg Asn Lys Trp Leu Leu Ile
   465                 470                 475                 480
   Ile Ile Leu Phe Val Glu Trp Leu Val Cys Ala Lys Tyr Gly Ala Arg
                   485                 490                 495
   Arg Tyr Ala Ala Gly Leu Pro Pro Pro Asp Ile Phe Val Pro Trp Ile
               500                 505                 510
   Val Ser Ser Ile Leu Phe Ser Leu Trp Cys Tyr Cys Tyr Tyr Thr Thr
           515                 520                 525
   Ala Asp Leu Arg Thr Asp Ser Gly Lys Gly Lys Leu Leu Ala Lys Ser
       530                 535                 540
   Pro Gly Gly Lys Gly Ala Gly Asp Glu Ser Ala Asn Leu Ala Ala Glu
   545                 550                 555                 560
   Ser Lys Met Thr Lys Arg Ser Ala Lys Gln Cys Pro Ala Ala Ser Trp
                   565                 570                 575
   Gln Asp Arg Ile Lys Leu Leu Val Leu Thr Leu Pro Pro Gln Ala Cys
               580                 585                 590
   Phe Met Pro Leu Leu Tyr Leu Ser Lys Phe Tyr Phe Phe Asp Tyr Val
           595                 600                 605
   Lys Ile Glu Arg Ser Gln
       610
<210> 3
   <211> 1884
   <212> DNA
   <213> Neospora caninum
<220>
   <221> CDS
   <222> (1)..(1884)
<400> 3
   atg caa ctc cgc gag aga aag ctc gcg gga gct ttt gac aac caa ggc      48
   Met Gln Leu Arg Glu Arg Lys Leu Ala Gly Ala Phe Asp Asn Gln Gly
   1               5                   10                  15
   tcg ccg cac cat ctg aag ccg cct gtg acg cct ggc tct tca tgc tcc      96
   Ser Pro His His Leu Lys Pro Pro Val Thr Pro Gly Ser Ser Cys Ser
               20                  25                  30
   tca gtc aac gag tgt tcg cgg cgg gcg tcc cat gac tcc aac gcg gcc     144
   Ser Val Asn Glu Cys Ser Arg Arg Ala Ser His Asp Ser Asn Ala Ala
           35                  40                  45
   tcg gtt tac cgc gaa cat gca gcc cac tat gcg cag aag ttt cga gcc     192
   Ser Val Tyr Arg Glu His Ala Ala His Tyr Ala Gln Lys Phe Arg Ala
       50                  55                  60
   gcc atc gac tac acg act cgg gag gaa cct cgc cag att ctg tgg ggt     240
   Ala Ile Asp Tyr Thr Thr Arg Glu Glu Pro Arg Gln Ile Leu Trp Gly
   65                  70                  75                  80
   ttt att ctt gtc ctg agc ttg ctt ctt ctc ggg ttc cgt gac cag ctg     288
   Phe Ile Leu Val Leu Ser Leu Leu Leu Leu Gly Phe Arg Asp Gln Leu
                   85                  90                  95
   ctg ccg tct tct tct cag gtg aac ctg gtg cac aga ggc gcc gga gaa         336
   Leu Pro Ser Ser Ser Gln Val Asn Leu Val His Arg Gly Ala Gly Glu
               100                 105                 110
   gct gtt cag ccc acg gtc tcg cca agt gag act gct gtc gct cct ggc         384
   Ala Val Gln Pro Thr Val Ser Pro Ser Glu Thr Ala Val Ala Pro Gly
           115                 120                 125
   gca aaa ctt cct gga aat gat gcc acg gtg aag tgc acc gcg ggt tcg         432
   Ala Lys Leu Pro Gly Asn Asp Ala Thr Val Lys Cys Thr Ala Gly Ser
       130                 135                 140
   cga aag aag aac gac aac gaa gat gtt gga gca gat ggg ggt gac gat         480
   Arg Lys Lys Asn Asp Asn Glu Asp Val Gly Ala Asp Gly Gly Asp Asp
   145                 150                 155                 160
   ccg aat gaa agc tgt cag cgg gct aca cca gag gct cct cgt gta tct         528
   Pro Asn Glu Ser Cys Gln Arg Ala Thr Pro Glu Ala Pro Arg Val Ser
                   165                 170                 175
   gag cag cag tct tct acg tcc ggt cgt act cag gtt gct cct tcg gtg         576
   Glu Gln Gln Ser Ser Thr Ser Gly Arg Thr Gln Val Ala Pro Ser Val
               180                 185                 190
   ttt tct tca atc ttt ggt ggt tcg tcc tcg gcc tct gtc tct tct cct         624
   Phe Ser Ser Ile Phe Gly Gly Ser Ser Ser Ala Ser Val Ser Ser Pro
           195                 200                 205
   ctg agc acg aat gtc agg cga gtg gtg act ggg ctt ctt gtc ctc gtc         672
   Leu Ser Thr Asn Val Arg Arg Val Val Thr Gly Leu Leu Val Leu Val
       210                 215                 220
   tgc ctg tac tgt ttc ctc cag gcg aaa gat ggt ttg ctc gtt cgt cca         720
   Cys Leu Tyr Cys Phe Leu Gln Ala Lys Asp Gly Leu Leu Val Arg Pro
   225                 230                 235                 240
   cac cct ggt ttc tgg cgc ctg gta cat ggc gtt tgc ctg gtt cac ttg         768
   His Pro Gly Phe Trp Arg Leu Val His Gly Val Cys Leu Val His Leu
                   245                 250                 255
   att gtt atg gtt gtg ctt ctg gtc ctc gac gtg gag acc gga cta ctt         816
   Ile Val Met Val Val Leu Leu Val Leu Asp Val Glu Thr Gly Leu Leu
               260                 265                 270
   gtt ttg gag ttg ttg ttc cca gaa att gca ggg aaa agg aaa gaa att         864
   Val Leu Glu Leu Leu Phe Pro Glu Ile Ala Gly Lys Arg Lys Glu Ile
           275                 280                 285
   ttt gca gga aca ctt gtg ctt gat tgc cga atc aac gga gac acg atc         912
   Phe Ala Gly Thr Leu Val Leu Asp Cys Arg Ile Asn Gly Asp Thr Ile
       290                 295                 300
   aag cgt cag tta acg agt gtg tgg ttc atc tcg cat gtt gtt ggt tgg         960
   Lys Arg Gln Leu Thr Ser Val Trp Phe Ile Ser His Val Val Gly Trp
   305                 310                 315                 320
   cta ggg aag atg gtc att ctc aga aat tgg ggt ctt tgt ctt ctt tac        1008
   Leu Gly Lys Met Val Ile Leu Arg Asn Trp Gly Leu Cys Leu Leu Tyr
                   325                 330                 335
   tcc atc ttc ttt gaa tta gga gaa ttg tcc ttc cac tgg ctt gtc cca        1056
   Ser Ile Phe Phe Glu Leu Gly Glu Leu Ser Phe His Trp Leu Val Pro
               340                 345                 350
   gaa ctt tgc gaa tgc tgg tgg gac agt atc ttc ata gat gct ctt ctg        1104
   Glu Leu Cys Glu Cys Trp Trp Asp Ser Ile Phe Ile Asp Ala Leu Leu
           355                 360                 365
   tcc aat gta agc ggc atg ctt ctg ggg gcc gtc ttc atg aag ttt atc        1152
   Ser Asn Val Ser Gly Met Leu Leu Gly Ala Val Phe Met Lys Phe Ile
       370                 375                 380
   aac atg cac cag tat gac tgg cta ggc cgc cat ccg ctg tac cag aag        1200
   Asn Met His Gln Tyr Asp Trp Leu Gly Arg His Pro Leu Tyr Gln Lys
   385                 390                 395                 400
   gtc ttc atg agt ttg act ccc ttt tct tcc gag ggc tat gac tgg agc        1248
   Val Phe Met Ser Leu Thr Pro Phe Ser Ser Glu Gly Tyr Asp Trp Ser
                   405                 410                 415
   ttc tac aag acc cca cgg cac ctg ttc ctg tcc atc gca ctg ctc acg        1296
   Phe Tyr Lys Thr Pro Arg His Leu Phe Leu Ser Ile Ala Leu Leu Thr
               420                 425                 430
   ttt tgc gtt ttc ttg gag ttg aat gtc ttc ttc ctt atg gcc gcg ctt        1344
   Phe Cys Val Phe Leu Glu Leu Asn Val Phe Phe Leu Met Ala Ala Leu
           435                 440                 445
   gat atc cct gca acc cac tat atc aac cct ctc cga acg ctg tac ctc        1392
   Asp Ile Pro Ala Thr His Tyr Ile Asn Pro Leu Arg Thr Leu Tyr Leu
       450                 455                 460
   act cta cta ggc gct gcg gcc gcc ccc gag cac tac gaa tat acc atg        1440
   Thr Leu Leu Gly Ala Ala Ala Ala Pro Glu His Tyr Glu Tyr Thr Met
   465                 470                 475                 480
   ttc agt cga gag aga gtt ggc cac aac gaa tgg tta ctt gtt gtg atc        1488
   Phe Ser Arg Glu Arg Val Gly His Asn Glu Trp Leu Leu Val Val Ile
                   485                 490                 495
   ctc agt gtc gaa ttg ttg gtg tgt gtc aag tat ggg gca ggc cgg tac        1536
   Leu Ser Val Glu Leu Leu Val Cys Val Lys Tyr Gly Ala Gly Arg Tyr
               500                 505                 510
   aac gcc gag acc cct ccc ctc gat cta gtt ata ccg tgg gtt gct gcg        1584
   Asn Ala Glu Thr Pro Pro Leu Asp Leu Val Ile Pro Trp Val Ala Ala
           515                 520                 525
   ctc agc ctc ttt gct gca tgg tgt tac tgt tac ttt tcg acg gct gac        1632
   Leu Ser Leu Phe Ala Ala Trp Cys Tyr Cys Tyr Phe Ser Thr Ala Asp
       530                 535                 540
   ctc cgc ggg aac aat ggc gag gaa ggg atc aag gcg cct gtg tcc cac        1680
   Leu Arg Gly Asn Asn Gly Glu Glu Gly Ile Lys Ala Pro Val Ser His
   545                 550                 555                 560
   gca cga aga ggg gga gaa gaa atg gaa aat ctg gca gaa gaa ccg aaa        1728
   Ala Arg Arg Gly Gly Glu Glu Met Glu Asn Leu Ala Glu Glu Pro Lys
                   565                 570                 575
   aag aca aaa cga tca gag aag cat cga gtg cat tcc tgg caa gac cag        1776
   Lys Thr Lys Arg Ser Glu Lys His Arg Val His Ser Trp Gln Asp Gln
               580                 585                 590
   gcc aag ctg ctt gtc att aca gtg cct cct cag gcg tgt ttt atc cct        1824
   Ala Lys Leu Leu Val Ile Thr Val Pro Pro Gln Ala Cys Phe Ile Pro
           595                 600                 605
   ctg ctg tat ctt atg aaa ttc tac ttt tac gac tac gtc aaa atc gag        1872
   Leu Leu Tyr Leu Met Lys Phe Tyr Phe Tyr Asp Tyr Val Lys Ile Glu
       610                 615                 620
   cgc aat cag tga                                                        1884
   Arg Asn Gln
   625
<210> 4
   <211> 627
   <212> PRT
   <213> Neospora caninum
<400> 4
   Met Gln Leu Arg Glu Arg Lys Leu Ala Gly Ala Phe Asp Asn Gln Gly
   1               5                   10                  15
   Ser Pro His His Leu Lys Pro Pro Val Thr Pro Gly Ser Ser Cys Ser
               20                  25                  30
   Ser Val Asn Glu Cys Ser Arg Arg Ala Ser His Asp Ser Asn Ala Ala
           35                  40                  45
   Ser Val Tyr Arg Glu His Ala Ala His Tyr Ala Gln Lys Phe Arg Ala
       50                  55                  60
   Ala Ile Asp Tyr Thr Thr Arg Glu Glu Pro Arg Gln Ile Leu Trp Gly
   65                  70                  75                  80
   Phe Ile Leu Val Leu Ser Leu Leu Leu Leu Gly Phe Arg Asp Gln Leu
                   85                  90                  95
   Leu Pro Ser Ser Ser Gln Val Asn Leu Val His Arg Gly Ala Gly Glu
               100                 105                 110
   Ala Val Gln Pro Thr Val Ser Pro Ser Glu Thr Ala Val Ala Pro Gly
           115                 120                 125
   Ala Lys Leu Pro Gly Asn Asp Ala Thr Val Lys Cys Thr Ala Gly Ser
       130                 135                 140
   Arg Lys Lys Asn Asp Asn Glu Asp Val Gly Ala Asp Gly Gly Asp Asp
   145                 150                 155                 160
   Pro Asn Glu Ser Cys Gln Arg Ala Thr Pro Glu Ala Pro Arg Val Ser
                   165                 170                 175
   Glu Gln Gln Ser Ser Thr Ser Gly Arg Thr Gln Val Ala Pro Ser Val
   180 185 190
   Phe Ser Ser Ile Phe Gly Gly Ser Ser Ser Ala Ser Val Ser Ser Pro
   195 200 205
   Leu Ser Thr Asn Val Arg Arg Val Val Thr Gly Leu Leu Val Leu Val
   210 215 220
   Cys Leu Tyr Cys Phe Leu Gln Ala Lys Asp Gly Leu Leu Val Arg Pro
   225 230 235 240
   His Pro Gly Phe Trp Arg Leu Val His Gly Val Cys Leu Val His Leu
   245 250 255
   Ile Val Met Val Val Leu Leu Val Leu Asp Val Glu Thr Gly Leu Leu
   260 265 270
   Val Leu Glu Leu Leu Phe Pro Glu Ile Ala Gly Lys Arg Lys Glu Ile
   275 280 285
   Phe Ala Gly Thr Leu Val Leu Asp Cys Arg Ile Asn Gly Asp Thr Ile
   290 295 300
   Lys Arg Gln Leu Thr Ser Val Trp Phe Ile Ser His Val Val Gly Trp
   305 310 315 320
   Leu Gly Lys Met Val Ile Leu Arg Asn Trp Gly Leu Cys Leu Leu Tyr
   325 330 335
   Ser Ile Phe Phe Glu Leu Gly Glu Leu Ser Phe His Trp Leu Val Pro
   340 345 350
   Glu Leu Cys Glu Cys Trp Trp Asp Ser Ile Phe Ile Asp Ala Leu Leu
   355 360 365
   Ser Asn Val Ser Gly Met Leu Leu Gly Ala Val Phe Met Lys Phe Ile
   370 375 380
   Asn Met His Gln Tyr Asp Trp Leu Gly Arg His Pro Leu Tyr Gln Lys
   385 390 395 400
   Val Phe Met Ser Leu Thr Pro Phe Ser Ser Glu Gly Tyr Asp Trp Ser
   405 410 415
   Phe Tyr Lys Thr Pro Arg His Leu Phe Leu Ser Ile Ala Leu Leu Thr
   420 425 430
   Phe Cys Val Phe Leu Glu Leu Asn Val Phe Phe Leu Met Ala Ala Leu
           435                 440                 445
   Asp Ile Pro Ala Thr His Tyr Ile Asn Pro Leu Arg Thr Leu Tyr Leu
       450                 455                 460
   Thr Leu Leu Gly Ala Ala Ala Ala Pro Glu His Tyr Glu Tyr Thr Met
   465                 470                 475                 480
   Phe Ser Arg Glu Arg Val Gly His Asn Glu Trp Leu Leu Val Val Ile
                   485                 490                 495
   Leu Ser Val Glu Leu Leu Val Cys Val Lys Tyr Gly Ala Gly Arg Tyr
               500                 505                 510
   Asn Ala Glu Thr Pro Pro Leu Asp Leu Val Ile Pro Trp Val Ala Ala
           515                 520                 525
   Leu Ser Leu Phe Ala Ala Trp Cys Tyr Cys Tyr Phe Ser Thr Ala Asp
       530                 535                 540
   Leu Arg Gly Asn Asn Gly Glu Glu Gly Ile Lys Ala Pro Val Ser His
   545                 550                 555                 560
   Ala Arg Arg Gly Gly Glu Glu Met Glu Asn Leu Ala Glu Glu Pro Lys
                   565                 570                 575
   Lys Thr Lys Arg Ser Glu Lys His Arg Val His Ser Trp Gln Asp Gln
               580                 585                 590
   Ala Lys Leu Leu Val Ile Thr Val Pro Pro Gln Ala Cys Phe Ile Pro
           595                 600                 605
   Leu Leu Tyr Leu Met Lys Phe Tyr Phe Tyr Asp Tyr Val Lys Ile Glu
       610                 615                 620
   Arg Asn Gln
   625
<210> 5
   <211> 1476
   <212> DNA
   <213> Eimeria tenella
<220>
   <221> CDS
   <222> (1)..(1476)
<400> 5
   atg cgg gtg agc cgc ggg cag gcg ccg acg ggg ggg ccc ggc ggg gcc       48
   Met Arg Val Ser Arg Gly Gln Ala Pro Thr Gly Gly Pro Gly Gly Ala
   1               5                   10                  15
   ccc gcg gcg ctg tcg ccg tgg ggg cgt ctg tgg gcc gct gcc tgg cgc       96
   Pro Ala Ala Leu Ser Pro Trp Gly Arg Leu Trp Ala Ala Ala Trp Arg
               20                  25                  30
   ccc gcg gcg gcg gcc gcc gct gct gct gcg gag ggg gcg gcc gct gcg      144
   Pro Ala Ala Ala Ala Ala Ala Ala Ala Ala Glu Gly Ala Ala Ala Ala
           35                  40                  45
   gcc gcg tcg ggc ggg ggg gcc tcg ggg gcc ccc cgc ggg cgg gca ggg      192
   Ala Ala Ser Gly Gly Gly Ala Ser Gly Ala Pro Arg Gly Arg Ala Gly
       50                  55                  60
   ggc ccc ccg ggg tgg ggg ctg ggg agc gtc ggg gcg ggg gcg ggg ctg      240
   Gly Pro Pro Gly Trp Gly Leu Gly Ser Val Gly Ala Gly Ala Gly Leu
   65                  70                  75                  80
   ctg ctg ctg ctg gcg ggc gcg ctg ccg ccg ctg ctg gcg agc tgg ggg      288
   Leu Leu Leu Leu Ala Gly Ala Leu Pro Pro Leu Leu Ala Ser Trp Gly
                   85                  90                  95
   ccg gcg gcg cag ggg ctg tcg gtg gcg gcg ctg ccg gcg gcg cag cag      336
   Pro Ala Ala Gln Gly Leu Ser Val Ala Ala Leu Pro Ala Ala Gln Gln
               100                 105                 110
   cag gag ctg cgg gcg ctg ctg ctg cgg agc ctg ggc tcg acg gcg ctg      384
   Gln Glu Leu Arg Ala Leu Leu Leu Arg Ser Leu Gly Ser Thr Ala Leu
           115                 120                 125
   ccg ctg tgc ctc tac ctc gcc ttc ctg ctg cag cag gcg cag gtg cag      432
   Pro Leu Cys Leu Tyr Leu Ala Phe Leu Leu Gln Gln Ala Gln Val Gln
       130                 135                 140
   cag ccg cag gcg cag cag gcg ctg gcg tgg agc gcg gtg cac ggc gcg      480
   Gln Pro Gln Ala Gln Gln Ala Leu Ala Trp Ser Ala Val His Gly Ala
   145                 150                 155                 160
   ggc gcg tgc tac ctg ctg ctg ctg ctg ctg ctg ctg gcg ctg ccc gcg      528
   Gly Ala Cys Tyr Leu Leu Leu Leu Leu Leu Leu Leu Ala Leu Pro Ala
                   165                 170                 175
   cgg ctc tgc cgc gaa ctg ctg cag ctg ctg ctg ccc ggc tgc tgc agc      576
   Arg Leu Cys Arg Glu Leu Leu Gln Leu Leu Leu Pro Gly Cys Cys Ser
               180                 185                 190
   ggc ggc aac gtc ttc acc ggc acg ctc atc ctc gac tgc tcg ctg cgc      624
   Gly Gly Asn Val Phe Thr Gly Thr Leu Ile Leu Asp Cys Ser Leu Arg
           195                 200                 205
   cgc agc acg ctg ctg cgc cag ctg cgc tcg ccc tgg ttc gcc agc cac      672
   Arg Ser Thr Leu Leu Arg Gln Leu Arg Ser Pro Trp Phe Ala Ser His
       210                 215                 220
   gcc ctc ggc tgg ttc ctc aag atg agc ctc tac cgc agc tgg ccc tgc      720
   Ala Leu Gly Trp Phe Leu Lys Met Ser Leu Tyr Arg Ser Trp Pro Cys
   225                 230                 235                 240
   gcc ctc gcg ctc tcc ctg ctt ttc gag gcc gca gag gct tcg ctg cac      768
   Ala Leu Ala Leu Ser Leu Leu Phe Glu Ala Ala Glu Ala Ser Leu His
                   245                 250                 255
   tgg ctg ctg ccc gag ttc cag gag tgc tgg tgg gac agc gtg gtg ctg      816
   Trp Leu Leu Pro Glu Phe Gln Glu Cys Trp Trp Asp Ser Val Val Leu
               260                 265                 270
   gac gcg gtt ctg tcg aac ctg ctg ggg atg act ccg cgg cac ttg gcc      864
   Asp Ala Val Leu Ser Asn Leu Leu Gly Met Thr Pro Arg His Leu Ala
           275                 280                 285
   atg acg gga ctg ctc ctc ggg ctc tcc ttg ctc gcg gag ctc aat gtt      912
   Met Thr Gly Leu Leu Leu Gly Leu Ser Leu Leu Ala Glu Leu Asn Val
       290                 295                 300
   ttc ttc ttg atg acg gcc ctg gac ctg cac gcc acg cac tgg gtc aac      960
   Phe Phe Leu Met Thr Ala Leu Asp Leu His Ala Thr His Trp Val Asn
   305                 310                 315                 320
   ccg ctc cgg ctg ctg ctc ctc ggg ctg ctg gcc ttc ccc gcg gtc gcg     1008
   Pro Leu Arg Leu Leu Leu Leu Gly Leu Leu Ala Phe Pro Ala Val Ala
                   325                 330                 335
   gag ttc tac gcc cag ctc cag cgc agc agc agc agc agc agc agc agc     1056
   Glu Phe Tyr Ala Gln Leu Gln Arg Ser Ser Ser Ser Ser Ser Ser Ser
               340                 345                 350
   agc ggc ggc ggc gtg ggg ccc aac gtc ttt ctt ttt gtc ttc att ttg     1104
   Ser Gly Gly Gly Val Gly Pro Asn Val Phe Leu Phe Val Phe Ile Leu
           355                 360                 365
   gcg gca gaa acc ctc gtt gct gcc aag tac ggc aga gac aga ttc cac     1152
   Ala Ala Glu Thr Leu Val Ala Ala Lys Tyr Gly Arg Asp Arg Phe His
       370                 375                 380
   tac aag agc ccc ccc aaa gat gtc ctg ctt ccc tgg atc ctc gct gct     1200
   Tyr Lys Ser Pro Pro Lys Asp Val Leu Leu Pro Trp Ile Leu Ala Ala
   385                 390                 395                 400
   gct ctc ttc gcc gcc tgg tgc tgc tcc tac ttc agc cag cag cag ggg     1248
   Ala Leu Phe Ala Ala Trp Cys Cys Ser Tyr Phe Ser Gln Gln Gln Gly
                   405                 410                 415
   ggc ccc cct ccc ccg ggg ggc ccc cag gcg cag ggg ggc ccc ccg ggg     1296
   Gly Pro Pro Pro Pro Gly Gly Pro Gln Ala Gln Gly Gly Pro Pro Gly
               420                 425                 430
   gag gac gcg gag aag gct tgc gag ggg gcg tgt ggg gtg ttt agg act     1344
   Glu Asp Ala Glu Lys Ala Cys Glu Gly Ala Cys Gly Val Phe Arg Thr
           435                 440                 445
   ttg aac aac ttc aaa cgg tct gct gcc aga ctg ctc ctc aaa atg ccg     1392
   Leu Asn Asn Phe Lys Arg Ser Ala Ala Arg Leu Leu Leu Lys Met Pro
       450                 455                 460
   ccc caa gcc tgc ttc att cct ctt ctt tac ttg gcc aaa aac tac tac     1440
   Pro Gln Ala Cys Phe Ile Pro Leu Leu Tyr Leu Ala Lys Asn Tyr Tyr
   465                 470                 475                 480
   tat gga gaa ccc cac acc cca cag cca gct ccc tag                     1476
   Tyr Gly Glu Pro His Thr Pro Gln Pro Ala Pro
                   485                 490
<210> 6
   <211> 491
   <212> PRT
   <213> Eimeria tenella
<400> 6
   Met Arg Val Ser Arg Gly Gln Ala Pro Thr Gly Gly Pro Gly Gly Ala
   1               5                   10                  15
   Pro Ala Ala Leu Ser Pro Trp Gly Arg Leu Trp Ala Ala Ala Trp Arg
               20                  25                  30
   Pro Ala Ala Ala Ala Ala Ala Ala Ala Ala Glu Gly Ala Ala Ala Ala
           35                  40                  45
   Ala Ala Ser Gly Gly Gly Ala Ser Gly Ala Pro Arg Gly Arg Ala Gly
       50                  55                  60
   Gly Pro Pro Gly Trp Gly Leu Gly Ser Val Gly Ala Gly Ala Gly Leu
   65                  70                  75                  80
   Leu Leu Leu Leu Ala Gly Ala Leu Pro Pro Leu Leu Ala Ser Trp Gly
                   85                  90                  95
   Pro Ala Ala Gln Gly Leu Ser Val Ala Ala Leu Pro Ala Ala Gln Gln
               100                 105                 110
   Gln Glu Leu Arg Ala Leu Leu Leu Arg Ser Leu Gly Ser Thr Ala Leu
           115                 120                 125
   Pro Leu Cys Leu Tyr Leu Ala Phe Leu Leu Gln Gln Ala Gln Val Gln
       130                 135                 140
   Gln Pro Gln Ala Gln Gln Ala Leu Ala Trp Ser Ala Val His Gly Ala
   145                 150                 155                 160
   Gly Ala Cys Tyr Leu Leu Leu Leu Leu Leu Leu Leu Ala Leu Pro Ala
                   165                 170                 175
   Arg Leu Cys Arg Glu Leu Leu Gln Leu Leu Leu Pro Gly Cys Cys Ser
               180                 185                 190
   Gly Gly Asn Val Phe Thr Gly Thr Leu Ile Leu Asp Cys Ser Leu Arg
           195                 200                 205
   Arg Ser Thr Leu Leu Arg Gln Leu Arg Ser Pro Trp Phe Ala Ser His
       210                 215                 220
   Ala Leu Gly Trp Phe Leu Lys Met Ser Leu Tyr Arg Ser Trp Pro Cys
   225 230 235 240
   Ala Leu Ala Leu Ser Leu Leu Phe Glu Ala Ala Glu Ala Ser Leu His
   245 250 255
   Trp Leu Leu Pro Glu Phe Gln Glu Cys Trp Trp Asp Ser Val Val Leu
   260 265 270
   Asp Ala Val Leu Ser Asn Leu Leu Gly Met Thr Pro Arg His Leu Ala
   275 280 285
   Met Thr Gly Leu Leu Leu Gly Leu Ser Leu Leu Ala Glu Leu Asn Val
   290 295 300
   Phe Phe Leu Met Thr Ala Leu Asp Leu His Ala Thr His Trp Val Asn
   305 310 315 320
   Pro Leu Arg Leu Leu Leu Leu Gly Leu Leu Ala Phe Pro Ala Val Ala
   325 330 335
   Glu Phe Tyr Ala Gln Leu Gln Arg Ser Ser Ser Ser Ser Ser Ser Ser
   340 345 350
   Ser Gly Gly Gly Val Gly Pro Asn Val Phe Leu Phe Val Phe Ile Leu
   355 360 365
   Ala Ala Glu Thr Leu Val Ala Ala Lys Tyr Gly Arg Asp Arg Phe His
   370 375 380
   Tyr Lys Ser Pro Pro Lys Asp Val Leu Leu Pro Trp Ile Leu Ala Ala
   385 390 395 400
   Ala Leu Phe Ala Ala Trp Cys Cys Ser Tyr Phe Ser Gln Gln Gln Gly
   405 410 415
   Gly Pro Pro Pro Pro Gly Gly Pro Gln Ala Gln Gly Gly Pro Pro Gly
   420 425 430
   Glu Asp Ala Glu Lys Ala Cys Glu Gly Ala Cys Gly Val Phe Arg Thr
   435 440 445
   Leu Asn Asn Phe Lys Arg Ser Ala Ala Arg Leu Leu Leu Lys Met Pro
   450 455 460
   Pro Gln Ala Cys Phe Ile Pro Leu Leu Tyr Leu Ala Lys Asn Tyr Tyr
   465 470 475 480
   Tyr Gly Glu Pro His Thr Pro Gln Pro Ala Pro
                   485                     490
<210> 7
   <211> 18
   <212> DNA
   <213> Artificial Sequence
<220>
   <223> Synthetic primer
<400> 7
   atgcaactcc cttcaaga 18
<210> 8
   <211> 25
   <212> DNA
   <213> Artificial Sequence
<220>
   <223> Synthetic primer
<400> 8
   tcactgactt cgttccattt tcacg 25
<210> 9
   <211> 20
   <212> DNA
   <213> Artificial Sequence
<220>
   <223> Synthetic primer
<400> 9
   atgtgtcggg gaccgccgct 20
<210> 10
   <211> 21
   <212> DNA
   <213> Artificial Sequence
<220>
   <223> Synthetic primer
<400> 10
   tcactcgtct ttttggcctt c 21
<210> 11
   <211> 32
   <212> DNA
   <213> Artificial Sequence
<220>
   <223> Synthetic primer
<400> 11
   ctcatcgcgg ccgcgttcgc ctcgagtgct tg 32
<210> 12
   <211> 30
   <212> DNA
   <213> Artificial Sequence
<220>
   <223> Synthetic primer
<400> 12
   ctcatcgaat tcacgagcca gtggaacgac 30
<210> 13
   <211> 32
   <212> DNA
   <213> Artificial Sequence
<220>
   <223> Synthetic primer
<400> 13
   ctcatcgtta acagcatctt tatcgatgcg ct 32
<210> 14
   <211> 34
   <212> DNA
   <213> Artificial Sequence
<220>
   <223> Synthetic primer
<400> 14
   ctcatcgtta actcactgac ttcgttcgat tttc 34
<210> 15
   <211> 20
   <212> DNA
   <213> Artificial Sequence
<220>
   <223> Synthetic primer
<400> 15
   cgattccttg agagcaactg 20
<210> 16
   <211> 20
   <212> DNA
   <213> Artificial Sequence
<220>
   <223> Synthetic primer
<400> 16
   gacgcagatg tgcgtgtatc 20
<210> 17
   <211> 21
   <212> DNA
   <213> Artificial Sequence
<220>
   <223> Synthetic primer
<400> 17
   actgccgtgt ggtaaaatga a 21
<210> 18
   <211> 23
   <212> DNA
   <213> Artificial Sequence
<220>
   <223> Synthetic primer
<400> 18
   gccatagagt tcattgcgga ctc 23
<210> 19
   <211> 21
   <212> DNA
   <213> Artificial Sequence
<220>
   <223> Synthetic primer
<400> 19
   tcccctctgc tggcgaaaag t 21
<210> 20
   <211> 25
   <212> DNA
   <213> Artificial Sequence
<220>
   <223> Synthetic primer
<400> 20
   agcgttcgtg gtcaactatc gattg 25
<210> 21
   <211> 24
   <212> DNA
   <213> Artificial Sequence
<220>
   <223> Synthetic primer
<400> 21
   tcttcgttag ctggcaactc acct 24
<210> 22
   <211> 24
   <212> DNA
   <213> Artificial Sequence
<220>
   <223> Synthetic primer
<400> 22
   atgacccagc agctaagcag atca 24
<210> 23
   <211> 46
   <212> PRT
   <213> Homo sapiens
<400> 23
   Arg Ser Tyr Gly Leu Cys Trp Thr Ile Ser Ile Thr Trp Glu Leu Thr
   1               5                   10                  15
   Glu Leu Phe Phe Met His Leu Leu Pro Asn Phe Ala Glu Cys Trp Trp
               20                  25                  30
   Asp Gln Val Ile Leu Asp Ile Leu Leu Cys Asn Gly Gly Gly
           35                  40                  45
<210> 24
   <211> 46
   <212> PRT
   <213> Cricetulus griseus
<400> 24
   Arg Ser Tyr Gly Leu Cys Trp Thr Ile Ser Ile Thr Trp Glu Leu Thr
   1               5                   10                  15
   Glu Leu Phe Phe Met His Leu Leu Pro Asn Phe Ala Glu Cys Trp Trp
               20                  25                  30
   Asp Gln Val Ile Leu Asp Ile Leu Leu Cys Asn Gly Gly Gly
           35                  40                  45
<210> 25
   <211> 46
   <212> PRT
   <213> Mus musculus
<400> 25
   Arg Ser Tyr Gly Leu Cys Trp Thr Ile Ser Ile Thr Trp Glu Leu Thr
   1               5                   10                  15
   Glu Leu Phe Phe Met His Leu Leu Pro Asn Phe Ala Glu Cys Trp Trp
               20                  25                  30
   Asp Gln Val Ile Leu Asp Ile Leu Leu Cys Asn Gly Gly Gly
           35                  40                  45
<210> 26
   <211> 46
   <212> PRT
   <213> Homo sapiens
<400> 26
   Arg Asp Trp Trp Met Cys Met Ile Ile Ser Val Met Phe Glu Phe Leu
   1               5                   10                  15
   Glu Tyr Ser Leu Glu His Gln Leu Pro Asn Phe Ser Glu Cys Trp Trp
               20                  25                  30
   Asp His Trp Ile Met Asp Val Leu Val Cys Asn Gly Leu Gly
           35                  40                  45
<210> 27
   <211> 46
   <212> PRT
   <213> Cricetulus griseus
<400> 27
   Arg Asp Trp Trp Met Cys Met Ile Ile Ser Val Met Phe Glu Phe Leu
   1               5                   10                  15
   Glu Tyr Ser Leu Glu His Gln Leu Pro Asn Phe Ser Glu Cys Trp Trp
               20                  25                  30
   Asp His Trp Ile Met Asp Val Leu Ile Cys Asn Gly Leu Gly
           35                  40                  45
<210> 28
   <211> 46
   <212> PRT
   <213> Mus musculus
<400> 28
   Arg Asp Trp Trp Met Cys Met Ile Ile Ser Val Met Phe Glu Phe Leu
   1               5                   10                  15
   Glu Tyr Ser Leu Glu His Gln Leu Pro Asn Phe Ser Glu Cys Trp Trp
               20                  25                  30
   Asp His Trp Ile Met Asp Val Leu Val Cys Asn Gly Leu Gly
           35                  40                  45
<210> 29
   <211> 46
   <212> PRT
   <213> Trypanosoma cruzi
<400> 29
   Arg Asp Trp Arg Phe Ala Thr Cys Val Ser Ile Thr Phe Glu Ile Ile
   1               5                   10                  15
   Glu Ile Thr Leu Gln His Ala Leu Pro Asn Phe Lys Glu Cys Trp Trp
               20                  25                  30
   Asp His Leu Leu Leu Asp Val Leu Leu Cys Asn Gly Gly Gly
           35                  40                  45
<210> 30
   <211> 46
   <212> PRT
   <213> Leishmania amazonensis
<400> 30
   Arg Asp Trp Arg Met Val Thr Ala Val Ser Leu Gly Phe Glu Val Val
   1               5                   10                  15
   Glu Val Thr Phe Gln His Val Leu Pro Asn Phe Ser Glu Cys Trp Trp
               20                  25                  30
   Asp His Ile Val Leu Asp Val Val Ile Cys Asn Ala Gly Gly
           35                  40                  45
<210> 31
   <211> 46
   <212> PRT
   <213> Cryptosporidium parvum
<400> 31
   Arg Asn Asn Phe Leu Val Trp Phe Asn Ser Ile Leu Phe Glu Trp Leu
   1               5                   10                  15
   Glu Ile Thr Leu Arg His Ile Leu Pro Asn Phe Tyr Glu Cys Trp Trp
               20                  25                  30
   Asp His Ile Ile Leu Asp Ile Phe Gly Cys Asn Met Ile Gly
           35                  40                  45
<210> 32
   <211> 46
   <212> PRT
   <213> Plasmodium falciparum
<400> 32
   Arg Asn Phe Phe Leu Leu Asn Ile Asn Ser Val Ile Phe Glu Leu Ile
   1               5                   10                  15
   Glu Leu Arg Phe Gln His Ile Leu Pro Asn Phe Tyr Glu Cys Trp Trp
   20 25 30
   Asp His Ile Phe Leu Asp Val Leu Ser Cys Asn Leu Ile Gly
   35 40 45
<210> 33
   <211> 46
   <212> PRT
   <213> Arabidopsis thaliana
<400> 33
   Arg Asn Gln Pro Leu Leu Trp Val Leu Ser Ile Gly Phe Glu Leu Leu
   1 5 10 15
   Glu Val Thr Phe Arg His Met Leu Pro Asn Phe Asn Glu Cys Trp Trp
   20 25 30
   Asp Ser Ile Val Leu Asp Ile Leu Ile Cys Asn Trp Phe Gly
   35 40 45
<210> 34
   <211> 46
   <212> PRT
   <213> Neospora caninum
<400> 34
   Arg Asp Ala Arg Leu Leu Trp Ile Leu Ser Leu Leu Phe Glu Trp Met
   1 5 10 15
   Glu Ile Ser Leu Arg His Ile Leu Pro Asn Phe Trp Glu Cys Trp Trp
   20 25 30
   Asp His Leu Ile Leu Asp Val Phe Gly Cys Asn Leu Leu Gly
   35 40 45
<210> 35
   <211> 46
   <212> PRT
   <213> Toxoplasma gondii
<400> 35
   Arg Asp Ala Arg Leu Leu Trp Ile Leu Ser Val Leu Phe Glu Trp Met
   1               5                   10                  15
   Glu Ile Ser Leu Arg His Ile Leu Pro Asn Phe Trp Glu Cys Trp Trp
               20                  25                  30
   Asp His Leu Ile Leu Asp Val Phe Gly Cys Asn Leu Leu Gly
           35                  40                  45
<210> 36
   <211> 46
   <212> PRT
   <213> Phytophthora infestans
<400> 36
   Arg Asp Trp Arg Leu Cys Trp Val Leu Ser Ile Ala Phe Glu Ile Leu
   1               5                   10                  15
   Glu Leu Ala Leu Gln Phe Val Ile Pro Asp Phe Gln Glu Cys Trp Trp
               20                  25                  30
   Asp Ser Leu Leu Leu Asp Leu Leu Gly Ala Asn Met Leu Gly
           35                  40                  45
<210> 37
   <211> 46
   <212> PRT
   <213> Perkinsus marinus
<400> 37
   Arg Asp Trp His Met Cys Trp Thr Tyr Ser Phe Ala Phe Glu Phe Ala
   1               5                   10                  15
   Glu Leu Gly Leu Val Trp Leu Val Pro Glu Phe Gln Glu Cys Trp Trp
               20                  25                  30
   Asp Ser Leu Leu Met Asp Val Phe Gly Ala Asn Phe Ile Gly
           35                  40                  45
<210> 38
   <211> 46
   <212> PRT
   <213> Eimeria tenella
<400> 38
   Arg Ser Trp Pro Cys Ala Leu Ala Leu Ser Leu Leu Phe Glu Ala Ala
   1               5                   10                  15
   Glu Ala Ser Leu His Trp Leu Leu Pro Glu Phe Gln Glu Cys Trp Trp
               20                  25                  30
   Asp Ser Val Val Leu Asp Ala Val Leu Ser Asn Leu Leu Gly
           35                  40                  45
<210> 39
   <211> 46
   <212> PRT
   <213> Neospora caninum
<400> 39
   Arg Asn Trp Gly Leu Cys Leu Leu Tyr Ser Ile Phe Phe Glu Leu Gly
   1               5                   10                  15
   Glu Leu Ser Phe His Trp Leu Val Pro Glu Leu Cys Glu Cys Trp Trp
               20                  25                  30
   Asp Ser Ile Phe Ile Asp Ala Leu Leu Ser Asn Val Ser Gly
           35                  40                  45
<210> 40
   <211> 46
   <212> PRT
   <213> Toxoplasma gondii
<400> 40
   Arg His Trp Gly Phe Cys Leu Ile Tyr Ser Leu Cys Phe Glu Leu Gly
   1               5                   10                  15
   Glu Leu Ser Phe His Trp Leu Val Pro Glu Leu Cys Glu Cys Trp Trp
               20                  25                  30
   Asp Ser Ile Phe Ile Asp Ala Leu Leu Ser Asn Val Cys Gly
           35                  40                  45